# EUROPEAN PATENT SPECIFICATION

(11) **EP 4 312 711 B1**
(45) Date of publication and mention of the grant of the patent: **09.12.2026**
(21) Application number: 22714812.9
(22) Date of filing: 14.03.2022
(51) Int. Cl.: A61B 1/00, A61B 1/06, A61B 1/04, A61B 1/313

(54) **AN IMAGE CAPTURE DEVICE, AN ENDOSCOPE SYSTEM AND A COMPUTER PROGRAM PRODUCT**
BILDERFASSUNGSVORRICHTUNG, ENDOSKOPSYSTEM UND COMPUTERPROGRAMMPRODUKT
DISPOSITIF DE CAPTURE D'IMAGE, SYSTÈME D'ENDOSCOPE ET PRODUIT PROGRAMME D'ORDINATEUR

(30) Priority: 30.03.2021 EP 21166062
(43) Date of publication of application: 07.02.2024
(73) Proprietor: Sony Semiconductor Solutions Corporation, Atsugi-shi, Kanagawa 243-0014 (JP); Sony Europe B.V., Weybridge, Surrey KT13 0XW (GB)
(72) Inventor: EMMERICH, Thimo, 70327 Stuttgart (DE); FACIUS, Zoltan, 70327 Stuttgart (DE); SPRINGER, Paul, 70327 Stuttgart (DE); SCHINZEL, Matthias, 70327 Stuttgart (DE); GATTO, Alexander, 70327 Stuttgart (DE)
(74) Representative: D Young & Co LLP
(86) International application number: PCT/EP2022/056553
(87) International publication number: WO 2022/207297

(56) References cited:
- JP-A- 2019 045 550
- JP-A- 2019 219 517
- US-A1- 2005 159 641
- US-A1- 2013 038 689
- US-A1- 2018 360 299
- US-A1- 2019 025 569
- US-A1- 2020 401 026

## Description

### BACKGROUND

The present invention relates to an image capture device, an endoscope system, an image capture method and a computer program product.

### Description of Related Art:

The "background" description provided herein is for the purpose of generally presenting the context of the disclosure. Work of the presently named inventors, to the extent it is described in the background section, as well as aspects of the description which may not otherwise qualify as prior art at the time of filing, are neither expressly or impliedly admitted as prior art against the present invention.

Image capture devices are used in a wide variety of situations in order to obtain images of a scene. The optical performance of the imaging device is thus a key factor in the quality of images and/or the amount of information regarding the scene which can be obtained.

One difficulty which is often encountered when using an image capture device is that it can be difficult to discriminate between different objects and/or surfaces within an image of a scene. This is a particular problem where the scene is complex (comprising a high number of different types of objects, for example) and/or where certain conditions place restrictions on the image capture device (such as restrictions on the size or form factor of the image capture device). In these situations, many different types of objects within a scene may appear very similar in the image and therefore it can be difficult to differentiate between these objects in the image which has been captured. Indeed, these problems are often exacerbated in endoscopic and/or laparoscopic imaging devices, which are often limited to small aperture diameters and/or form factors.

In particular, in a surgical environment, it is of high importance for a surgeon to be able to discriminate between different types of tissue or different types of anatomical structures when performing a surgical procedure or during a surgical intervention. Moreover, owing to the nature of the surgical environment, it is often required that such discrimination between the different types of tissue or different types of anatomical structures can be realised in a substantially real time environment. This further exacerbates the issues with existing image capture devices.

Current systems which can be used to provide a partial increase in the differentiation between objects in a scene have the disadvantage of relatively large size, high complexity and no real-time capability from acquisition and image processing point of view.

It is an aim of the present disclosure to address these issues.

Prior art includes: US 2019/025569 A1, US 2013/038689 A1, US 2018/360299 A1, JP 2019 219517 A. Each of these prior art disclosures describes a prior art device.

Indeed, US 2019/025569 A1 discloses an image capture unit includes a prism assembly and sensor assembly. The prism assembly includes a beam splitter, while the sensor assembly includes coplanar image capture sensors. Each of the coplanar image capture sensors has a common front end optical structure, e.g., the optical structure distal to the image capture unit is the same for each of the sensors. A controller enhances images acquired by the coplanar image capture sensors. The enhanced images may include (a) visible images with enhanced feature definition, in which a particular feature in the scene is emphasized to the operator of minimally invasive surgical system; (b) images having increased image apparent resolution; (c) images having increased dynamic range; (d) images displayed in a way based on a pixel color component vector having three or more color components; and (e) images having extended depth of field.

### SUMMARY

The invention is defined by the appended claims.

The embodiments of the present disclosure provide an image capture device which is able to provide an improved accuracy and robustness of discrimination between objects within the scene in a substantially real time environment.

Of course, the present disclosure is not particularly limited to these advantageous technical effects, there may be others as will be apparent to the skilled person when reading the disclosure.

The foregoing paragraphs have been provided by way of general introduction, and are not intended to limit the scope of the following claims. The described embodiments, together with further advantages, will be best understood by reference to the following detailed description taken in conjunction with the accompanying drawings.

### BRIEF DESCRIPTION OF THE DRAWINGS

A more complete appreciation of the disclosure and many of the attendant advantages thereof will be readily obtained as the same becomes better understood by reference to the following detailed description when considered in connection with the accompanying drawings, wherein:
Figure 1 is a view depicting an example of a schematic configuration of an endoscopic surgery system to which the technology according to an embodiment of the present disclosure can be applied;
Figure 2 is a block diagram depicting an example of a functional configuration of the camera head and the CCU depicted in Figure 1;
Figure 3 illustrates an example configuration of an image capture device in accordance with embodiments of the disclosure;
Figure 4 illustrates an example configuration of a beam splitter in accordance with embodiments of the disclosure;
Figure 5A illustrates an example configuration of image sensor circuitry in accordance with embodiments of the disclosure;
Figure 5B illustrates an example configuration of a filter unit in accordance with embodiments of the disclosure.
Figure 6 illustrates an example configuration of an endoscope system in accordance with embodiments of the disclosure;
Figure 7 illustrates an example configuration of an image capture device in accordance with embodiments of the disclosure;
Figure 8 illustrates an example configuration of an image capture device in accordance with embodiments of the disclosure;
Figure 9 illustrates a process of image acquisition in accordance with embodiments of the disclosure;
Figure 10 illustrates a computer device in accordance with embodiments of the disclosure;
Figure 11 illustrates an image capture method in accordance with embodiments of the disclosure.

### DESCRIPTION OF THE EMBODIMENTS

Referring now to the drawings, wherein like reference numerals designate identical or corresponding parts throughout the several views.

### <<Application>>

The technology according to an embodiment of the present disclosure can be applied to various products. For example, the technology according to an embodiment of the present disclosure may be applied to an endoscopic surgery system, surgical microscopy or medical imaging device or other kind of industrial endoscopy in, say pipe or tube laying or fault finding.

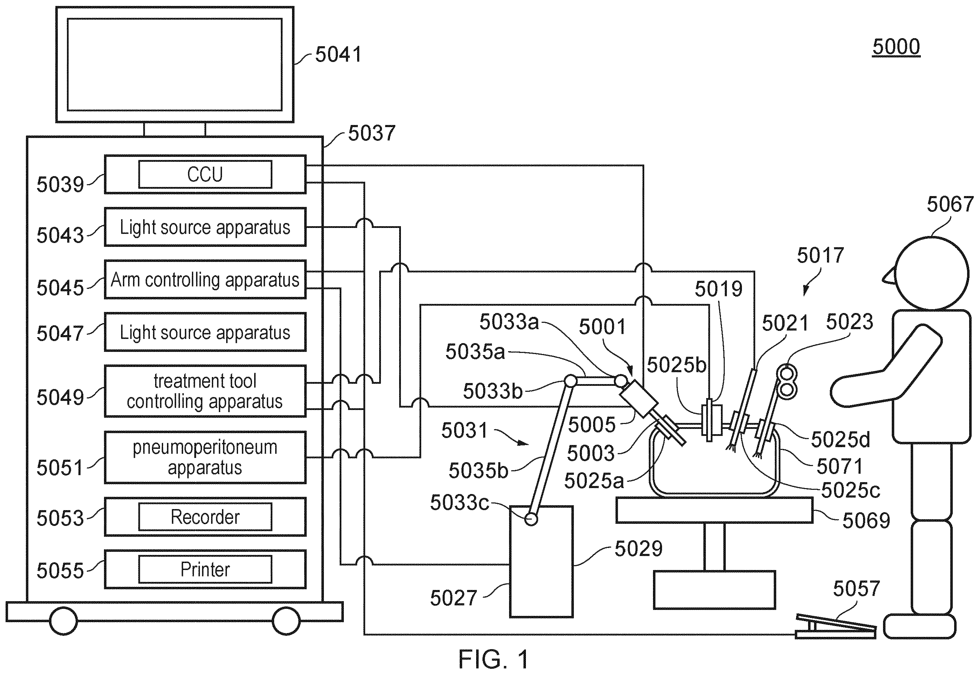

Figure 1 is a view depicting an example of a schematic configuration of an endoscopic surgery system 5000 to which the technology according to an embodiment of the present disclosure can be applied. In Figure 1, a state is illustrated in which a surgeon (medical doctor) 5067 is using the endoscopic surgery system 5000 to perform surgery for a patient 5071 on a patient bed 5069. As depicted, the endoscopic surgery system 5000 includes an endoscope 5001, other surgical tools 5017, a supporting arm apparatus 5027 which supports the endoscope 5001 thereon, and a cart 5037 on which various apparatus for endoscopic surgery are mounted.

In endoscopic surgery, in place of incision of the abdominal wall to perform laparotomy, a plurality of tubular aperture devices called trocars 5025a to 5025d are used to puncture the abdominal wall. Then, a lens barrel 5003 of the endoscope 5001 and the other surgical tools 5017 are inserted into body lumens of the patient 5071 through the trocars 5025a to 5025d. In the example depicted, as the other surgical tools 5017, a pneumoperitoneum tube 5019, an energy treatment tool 5021 and forceps 5023 are inserted into body lumens of the patient 5071. Further, the energy treatment tool 5021 is a treatment tool for performing incision and peeling of a tissue, sealing of a blood vessel or the like by high frequency current or ultrasonic vibration. However, the surgical tools 5017 depicted are mere examples at all, and as the surgical tools 5017, various surgical tools which are generally used in endoscopic surgery such as, for example, a pair of tweezers or a retractor may be used.

An image of a surgical region in a body lumen of the patient 5071 imaged by the endoscope 5001 is displayed on a display apparatus 5041. The surgeon 5067 would use the energy treatment tool 5021 or the forceps 5023 while watching the image of the surgical region displayed on the display apparatus 5041 on the real time basis to perform such treatment as, for example, resection of an affected area. It is to be noted that, though not depicted, the pneumoperitoneum tube 5019, the energy treatment tool 5021 and the forceps 5023 are supported by the surgeon 5067, an assistant or the like during surgery.

### (Supporting Arm Apparatus)

The supporting arm apparatus 5027 includes an arm unit 5031 extending from a base unit 5029. In the example depicted, the arm unit 5031 includes joint portions 5033a, 5033b and 5033c and links 5035a and 5035b and is driven under the control of an arm controlling apparatus 5045. The endoscope 5001 is supported by the arm unit 5031 such that the position and the posture of the endoscope 5001 are controlled. Consequently, stable fixation in position of the endoscope 5001 can be implemented.

### (Endoscope)

The endoscope 5001 includes the lens barrel 5003 which has a region of a predetermined length from a distal end thereof to be inserted into a body lumen of the patient 5071, and a camera head 5005 connected to a proximal end of the lens barrel 5003. In the example depicted, the endoscope 5001 is depicted which includes as a hard mirror having the lens barrel 5003 of the hard type. However, the endoscope 5001 may otherwise be configured as a soft mirror having the lens barrel 5003 of the soft type.

The lens barrel 5003 has, at a distal end thereof, an opening in which an objective lens is fitted. A light source apparatus 5043 is connected to the endoscope 5001 such that light generated by the light source apparatus 5043 is introduced to a distal end of the lens barrel by a light guide extending in the inside of the lens barrel 5003 and is irradiated toward an observation target in a body lumen of the patient 5071 through the objective lens. It is to be noted that the endoscope 5001 may be a direct view mirror or may be a perspective view mirror or a side view mirror.

An optical system and an image pickup element are provided in the inside of the camera head 5005 such that reflected light (observation light) from an observation target is condensed on the image pickup element by the optical system. The observation light is photo-electrically converted by the image pickup element to generate an electric signal corresponding to the observation light, namely, an image signal corresponding to an observation image. The image signal is transmitted as RAW data to a CCU 5039. It is to be noted that the camera head 5005 has a function incorporated therein for suitably driving the optical system of the camera head 5005 to adjust the magnification and the focal distance.

It is to be noted that, in order to establish compatibility with, for example, a stereoscopic vision (three dimensional (3D) display), a plurality of image pickup elements may be provided on the camera head 5005. In this case, a plurality of relay optical systems are provided in the inside of the lens barrel 5003 in order to guide observation light to each of the plurality of image pickup elements.

### (Various Apparatus Incorporated in Cart)

The CCU 5039 includes a central processing unit (CPU), a graphics processing unit (GPU) or the like and integrally controls operation of the endoscope 5001 and the display apparatus 5041. In particular, the CCU 5039 performs, for an image signal received from the camera head 5005, various image processes for displaying an image based on the image signal such as, for example, a development process (demosaic process). The CCU 5039 provides the image signal for which the image processes have been performed to the display apparatus 5041. Further, the CCU 5039 transmits a control signal to the camera head 5005 to control driving of the camera head 5005. The control signal may include information relating to an image pickup condition such as a magnification or a focal distance.

The display apparatus 5041 displays an image based on an image signal for which the image processes have been performed by the CCU 5039 under the control of the CCU 5039. If the endoscope 5001 is ready for imaging of a high resolution such as 4K (horizontal pixel number 3840 × vertical pixel number 2160), 8K (horizontal pixel number 7680 × vertical pixel number 4320) or the like and/or ready for 3D display, then a display apparatus by which corresponding display of the high resolution and/or 3D display are possible may be used as the display apparatus 5041. Where the apparatus is ready for imaging of a high resolution such as 4K or 8K, if the display apparatus used as the display apparatus 5041 has a size of equal to or not less than 55 inches, then a more immersive experience can be obtained. Further, a plurality of display apparatus 5041 having different resolutions and/or different sizes may be provided in accordance with purposes.

The light source apparatus 5043 includes a light source such as, for example, a light emitting diode (LED) and supplies irradiation light for imaging of a surgical region to the endoscope 5001.

The arm controlling apparatus 5045 includes a processor such as, for example, a CPU and operates in accordance with a predetermined program to control driving of the arm unit 5031 of the supporting arm apparatus 5027 in accordance with a predetermined controlling method.

An inputting apparatus 5047 is an input interface for the endoscopic surgery system 5000. A user can perform inputting of various kinds of information or instruction inputting to the endoscopic surgery system 5000 through the inputting apparatus 5047. For example, the user would input various kinds of information relating to surgery such as physical information of a patient, information regarding a surgical procedure of the surgery and so forth through the inputting apparatus 5047. Further, the user would input, for example, an instruction to drive the arm unit 5031, an instruction to change an image pickup condition (type of irradiation light, magnification, focal distance or the like) by the endoscope 5001, an instruction to drive the energy treatment tool 5021 or the like through the inputting apparatus 5047.

The type of the inputting apparatus 5047 is not limited and may be that of any one of various known inputting apparatus. As the inputting apparatus 5047, for example, a mouse, a keyboard, a touch panel, a switch, a foot switch 5057 and/or a lever or the like may be applied. Where a touch panel is used as the inputting apparatus 5047, it may be provided on the display face of the display apparatus 5041.

Otherwise, the inputting apparatus 5047 is a device to be mounted on a user such as, for example, a glasses type wearable device or a head mounted display (HMD), and various kinds of inputting are performed in response to a gesture or a line of sight of the user detected by any of the devices mentioned. Further, the inputting apparatus 5047 includes a camera which can detect a motion of a user, and various kinds of inputting are performed in response to a gesture or a line of sight of a user detected from a video imaged by the camera. Further, the inputting apparatus 5047 includes a microphone which can collect the voice of a user, and various kinds of inputting are performed by voice collected by the microphone. By configuring the inputting apparatus 5047 such that various kinds of information can be inputted in a contactless fashion in this manner, especially a user who belongs to a clean area (for example, the surgeon 5067) can operate an apparatus belonging to an unclean area in a contactless fashion. Further, since the user can operate an apparatus without releasing a possessed surgical tool from its hand, the convenience to the user is improved.

A treatment tool controlling apparatus 5049 controls driving of the energy treatment tool 5021 for cautery or incision of a tissue, sealing of a blood vessel or the like. A pneumoperitoneum apparatus 5051 feeds gas into a body lumen of the patient 5071 through the pneumoperitoneum tube 5019 to inflate the body lumen in order to secure the field of view of the endoscope 5001 and secure the working space for the surgeon. A recorder 5053 is an apparatus capable of recording various kinds of information relating to surgery. A printer 5055 is an apparatus capable of printing various kinds of information relating to surgery in various forms such as a text, an image or a graph.

In the following, especially a characteristic configuration of the endoscopic surgery system 5000 is described in more detail.

### (Supporting Arm Apparatus)

The supporting arm apparatus 5027 includes the base unit 5029 serving as a base, and the arm unit 5031 extending from the base unit 5029. In the example depicted, the arm unit 5031 includes the plurality of joint portions 5033a, 5033b and 5033c and the plurality of links 5035a and 5035b connected to each other by the joint portion 5033b. In Figure 1, for simplified illustration, the configuration of the arm unit 5031 is depicted in a simplified form. Actually, the shape, number and arrangement of the joint portions 5033a to 5033c and the links 5035a and 5035b and the direction and so forth of axes of rotation of the joint portions 5033a to 5033c can be set suitably such that the arm unit 5031 has a desired degree of freedom. For example, the arm unit 5031 may preferably be configured such that it has a degree of freedom equal to or not less than 6 degrees of freedom. This makes it possible to move the endoscope 5001 freely within the movable range of the arm unit 5031. Consequently, it becomes possible to insert the lens barrel 5003 of the endoscope 5001 from a desired direction into a body lumen of the patient 5071.

An actuator is provided in each of the joint portions 5033a to 5033c, and the joint portions 5033a to 5033c are configured such that they are rotatable around predetermined axes of rotation thereof by driving of the respective actuators. The driving of the actuators is controlled by the arm controlling apparatus 5045 to control the rotational angle of each of the joint portions 5033a to 5033c thereby to control driving of the arm unit 5031. Consequently, control of the position and the posture of the endoscope 5001 can be implemented. Thereupon, the arm controlling apparatus 5045 can control driving of the arm unit 5031 by various known controlling methods such as force control or position control.

For example, if the surgeon 5067 suitably performs operation inputting through the inputting apparatus 5047 (including the foot switch 5057), then driving of the arm unit 5031 may be controlled suitably by the arm controlling apparatus 5045 in response to the operation input to control the position and the posture of the endoscope 5001. After the endoscope 5001 at the distal end of the arm unit 5031 is moved from an arbitrary position to a different arbitrary position by the control just described, the endoscope 5001 can be supported fixedly at the position after the movement. It is to be noted that the arm unit 5031 may be operated in a master-slave fashion. In this case, the arm unit 5031 may be remotely controlled by the user through the inputting apparatus 5047 which is placed at a place remote from the surgery room.

Further, where force control is applied, the arm controlling apparatus 5045 may perform power-assisted control to drive the actuators of the joint portions 5033a to 5033c such that the arm unit 5031 may receive external force by the user and move smoothly following the external force. This makes it possible to move, when the user directly touches with and moves the arm unit 5031, the arm unit 5031 with comparatively weak force. Accordingly, it becomes possible for the user to move the endoscope 5001 more intuitively by a simpler and easier operation, and the convenience to the user can be improved.

Here, generally in endoscopic surgery, the endoscope 5001 is supported by a medical doctor called scopist. In contrast, where the supporting arm apparatus 5027 is used, the position of the endoscope 5001 can be fixed more certainly without hands, and therefore, an image of a surgical region can be obtained stably and surgery can be performed smoothly.

It is to be noted that the arm controlling apparatus 5045 may not necessarily be provided on the cart 5037. Further, the arm controlling apparatus 5045 may not necessarily be a single apparatus. For example, the arm controlling apparatus 5045 may be provided in each of the joint portions 5033a to 5033c of the arm unit 5031 of the supporting arm apparatus 5027 such that the plurality of arm controlling apparatus 5045 cooperate with each other to implement driving control of the arm unit 5031.

### (Light Source Apparatus)

The light source apparatus 5043 supplies irradiation light upon imaging of a surgical region to the endoscope 5001. The light source apparatus 5043 includes a white light source which includes, for example, an LED, a laser light source or a combination of them. In this case, where a white light source includes a combination of red, green, and blue (RGB) laser light sources, since the output intensity and the output timing can be controlled with a high degree of accuracy for each colour (each wavelength), adjustment of the white balance of a picked up image can be performed by the light source apparatus 5043. Further, in this case, if laser beams from the respective RGB laser light sources are irradiated time-divisionally on an observation target and driving of the image pickup elements of the camera head 5005 is controlled in synchronism with the irradiation timings, then images individually corresponding to the R, G and B colours can be picked up time-divisionally.

According to the method just described, a colour image can be obtained even if a colour filter is not provided for the image pickup element.

Further, driving of the light source apparatus 5043 may be controlled such that the intensity of light to be outputted is changed for each predetermined time. By controlling driving of the image pickup element of the camera head 5005 in synchronism with the timing of the change of the intensity of light to acquire images time-divisionally and synthesizing the images, an image of a high dynamic range free from underexposed blocked up shadows and overexposed highlights can be created.

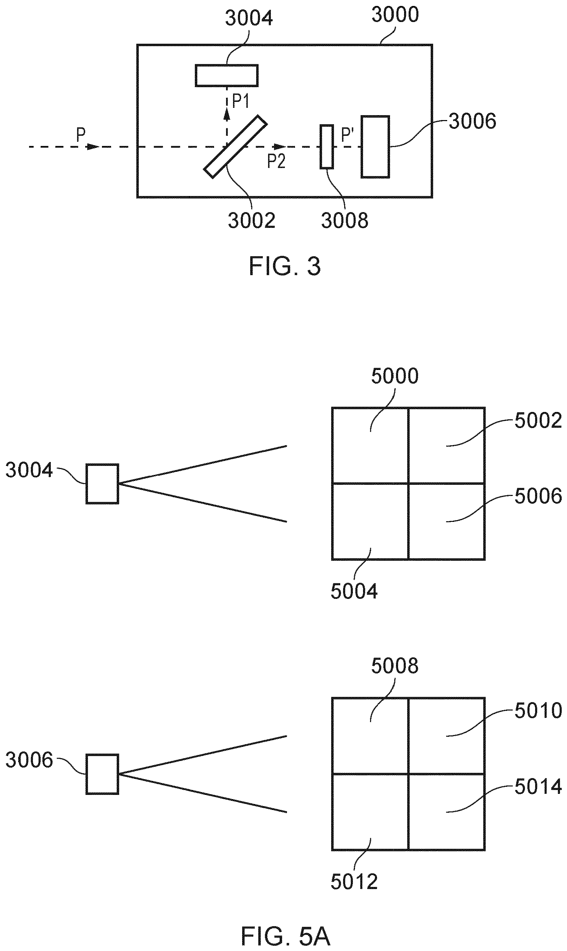

Further, the light source apparatus 5043 may be configured to supply light of a predetermined wavelength band ready for special light observation. This may include, but not be limited to laser light such as that provided by a Vertical Cavity surface laser or any kind of laser light. Alternatively or additionally, the light may be InfraRed (IR) light. In special light observation, for example, by utilizing the wavelength dependency of absorption of light in a body tissue to irradiate light of a narrower band in comparison with irradiation light upon ordinary observation (namely, white light), narrow band light observation (narrow band imaging) of imaging a predetermined tissue such as a blood vessel of a superficial portion of the mucous membrane or the like in a high contrast is performed. Alternatively, in special light observation, fluorescent observation for obtaining an image from fluorescent light generated by irradiation of excitation light may be performed. In fluorescent observation, it is possible to perform observation of fluorescent light from a body tissue by irradiating excitation light on the body tissue (autofluorescence observation) or to obtain a fluorescent light image by locally injecting a reagent such as indocyanine green (ICG) into a body tissue and irradiating excitation light corresponding to a fluorescent light wavelength of the reagent upon the body tissue. The light source apparatus 5043 can be configured to supply such narrow-band light and/or excitation light suitable for special light observation as described above. The light source may also apply a heat pattern to an area. This heat pattern will be explained later with reference to Figures 3A-C. The light source apparatus 5043 is, in embodiments, a Vertical Cavity Surface-Emitting Laser (VCSEL) which can produce light in the visible part of the electromagnetic spectrum and some produce light in the Infra-Red part of the electromagnetic spectrum. In this respect, the light source apparatus 5043 may also act as a visible light source illuminating the area. The light source apparatus 5043 is, in embodiments, one or more Vertical Cavity Surface-Emitting Laser (VCSEL) which can produce light in the visible part of the electromagnetic spectrum and some produce light in the Infra-Red part of the electromagnetic spectrum. In this respect, the light source apparatus 5043 may also act as a visible light source illuminating the area. The one or more VCSELs may be single wavelength narrowband VCSELs, where each VCSEL varies in emission spectral frequency. Alternatively, or additionally, one or more of the VCSELs may be a Micro Electro Mechanical system (MEMs) type VCSEL whose wavelength emission may be altered over a specific range. In embodiments of the disclosure, the wavelength may alter over the range 550nm to 650nm or 600nm to 650nm. The shape of the VCSEL may vary such as a square or circular shape and may be positioned at one or varying positions in the endoscope 5001.

The light source apparatus 5043 may illuminate one or more areas. This may be achieved by selectively switching the VCSELs on or by performing a raster scan of the area using a Micro Electro Mechanical system (MEMs). The purpose of the light source apparatus 5043 is to perform Spatial Light Modulation (SLM) on the light over the area. This will be explained in more detail later.

It should be noted that although the foregoing describes the light source apparatus 5043 as being positioned in the cart, the disclosure is not so limited. In particular, the light source apparatus may be positioned in the camera head 5005.

### (Camera Head and CCU)

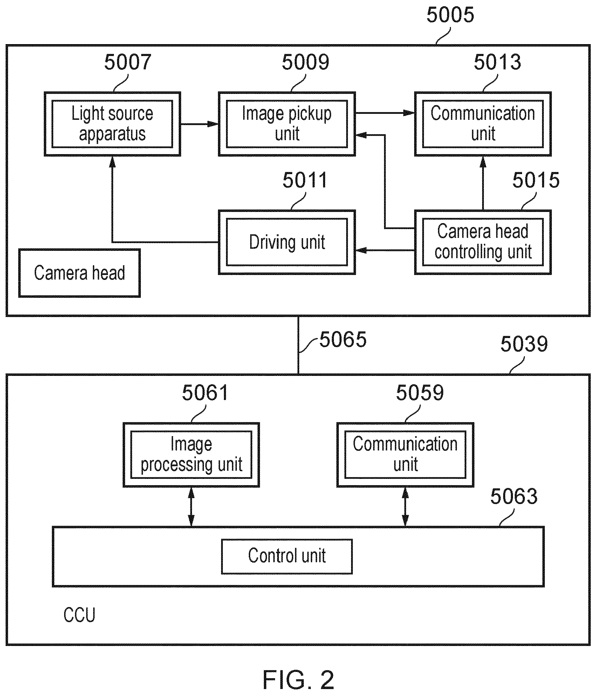

Functions of the camera head 5005 of the endoscope 5001 and the CCU 5039 are described in more detail with reference to Figure 2. Figure 2 is a block diagram depicting an example of a functional configuration of the camera head 5005 and the CCU 5039 depicted in Figure 1.

Referring to Figure 2, the camera head 5005 has, as functions thereof, a lens unit 5007, an image pickup unit 5009, a driving unit 5011, a communication unit 5013 and a camera head controlling unit 5015. Further, the CCU 5039 has, as functions thereof, a communication unit 5059, an image processing unit 5061 and a control unit 5063. The camera head 5005 and the CCU 5039 are connected to be bidirectionally communicable to each other by a transmission cable 5065.

First, a functional configuration of the camera head 5005 is described. The lens unit 5007 is an optical system provided at a connecting location of the camera head 5005 to the lens barrel 5003.

Observation light taken in from a distal end of the lens barrel 5003 is introduced into the camera head 5005 and enters the lens unit 5007. The lens unit 5007 includes a combination of a plurality of lenses including a zoom lens and a focusing lens. The lens unit 5007 has optical properties adjusted such that the observation light is condensed on a light receiving face of the image pickup element of the image pickup unit 5009. Further, the zoom lens and the focusing lens are configured such that the positions thereof on their optical axis are movable for adjustment of the magnification and the focal point of a picked up image.

The image pickup unit 5009 includes an image pickup element and disposed at a succeeding stage to the lens unit 5007. Observation light having passed through the lens unit 5007 is condensed on the light receiving face of the image pickup element, and an image signal corresponding to the observation image is generated by photoelectric conversion of the image pickup element. The image signal generated by the image pickup unit 5009 is provided to the communication unit 5013.

As the image pickup element which is included by the image pickup unit 5009, an image sensor, for example, of the complementary metal oxide semiconductor (CMOS) type is used which has a Bayer array and is capable of picking up an image in colour. It is to be noted that, as the image pickup element, an image pickup element may be used which is ready, for example, for imaging of an image of a high resolution equal to or not less than 4K. If an image of a surgical region is obtained in a high resolution, then the surgeon 5067 can comprehend a state of the surgical region in enhanced details and can proceed with the surgery more smoothly.

Further, the image pickup element which is included by the image pickup unit 5009 includes such that it has a pair of image pickup elements for acquiring image signals for the right eye and the left eye compatible with 3D display. Where 3D display is applied, the surgeon 5067 can comprehend the depth of a living body tissue in the surgical region more accurately. It is to be noted that, if the image pickup unit 5009 is configured as that of the multi-plate type, then a plurality of systems of lens units 5007 are provided corresponding to the individual image pickup elements of the image pickup unit 5009.

The image pickup unit 5009 may not necessarily be provided on the camera head 5005. For example, the image pickup unit 5009 may be provided just behind the objective lens in the inside of the lens barrel 5003.

The driving unit 5011 includes an actuator and moves the zoom lens and the focusing lens of the lens unit 5007 by a predetermined distance along the optical axis under the control of the camera head controlling unit 5015. Consequently, the magnification and the focal point of a picked up image by the image pickup unit 5009 can be adjusted suitably.

The communication unit 5013 includes a communication apparatus for transmitting and receiving various kinds of information to and from the CCU 5039. The communication unit 5013 transmits an image signal acquired from the image pickup unit 5009 as RAW data to the CCU 5039 through the transmission cable 5065. Thereupon, in order to display a picked up image of a surgical region in low latency, preferably the image signal is transmitted by optical communication. This is because, upon surgery, the surgeon 5067 performs surgery while observing the state of an affected area through a picked up image, it is demanded for a moving image of the surgical region to be displayed on the real time basis as far as possible in order to achieve surgery with a higher degree of safety and certainty. Where optical communication is applied, a photoelectric conversion module for converting an electric signal into an optical signal is provided in the communication unit 5013. After the image signal is converted into an optical signal by the photoelectric conversion module, it is transmitted to the CCU 5039 through the transmission cable 5065.

Further, the communication unit 5013 receives a control signal for controlling driving of the camera head 5005 from the CCU 5039. The control signal includes information relating to image pickup conditions such as, for example, information that a frame rate of a picked up image is designated, information that an exposure value upon image picking up is designated and/or information that a magnification and a focal point of a picked up image are designated. The communication unit 5013 provides the received control signal to the camera head controlling unit 5015. It is to be noted that also the control signal from the CCU 5039 may be transmitted by optical communication. In this case, a photoelectric conversion module for converting an optical signal into an electric signal is provided in the communication unit 5013. After the control signal is converted into an electric signal by the photoelectric conversion module, it is provided to the camera head controlling unit 5015.

It is to be noted that the image pickup conditions such as the frame rate, exposure value, magnification or focal point are set automatically by the control unit 5063 of the CCU 5039 on the basis of an acquired image signal. In other words, an auto exposure (AE) function, an auto focus (AF) function and an auto white balance (AWB) function are incorporated in the endoscope 5001.

The camera head controlling unit 5015 controls driving of the camera head 5005 on the basis of a control signal from the CCU 5039 received through the communication unit 5013. For example, the camera head controlling unit 5015 controls driving of the image pickup element of the image pickup unit 5009 on the basis of information that a frame rate of a picked up image is designated and/or information that an exposure value upon image picking up is designated. Further, for example, the camera head controlling unit 5015 controls the driving unit 5011 to suitably move the zoom lens and the focus lens of the lens unit 5007 on the basis of information that a magnification and a focal point of a picked up image are designated. The camera head controlling unit 5015 may further include a function for storing information for identifying the lens barrel 5003 and/or the camera head 5005.

It is to be noted that, by disposing the components such as the lens unit 5007 and the image pickup unit 5009 in a sealed structure having high airtightness and waterproof, the camera head 5005 can be provided with resistance to an autoclave sterilization process.

Now, a functional configuration of the CCU 5039 is described. The communication unit 5059 includes a communication apparatus for transmitting and receiving various kinds of information to and from the camera head 5005. The communication unit 5059 receives an image signal transmitted thereto from the camera head 5005 through the transmission cable 5065. Thereupon, the image signal may be transmitted preferably by optical communication as described above. In this case, for the compatibility with optical communication, the communication unit 5059 includes a photoelectric conversion module for converting an optical signal into an electric signal. The communication unit 5059 provides the image signal after conversion into an electric signal to the image processing unit 5061.

Further, the communication unit 5059 transmits, to the camera head 5005, a control signal for controlling driving of the camera head 5005. The control signal may also be transmitted by optical communication.

The image processing unit 5061 performs various image processes for an image signal in the form of RAW data transmitted thereto from the camera head 5005. The image processes include various known signal processes such as, for example, a development process, an image quality improving process (a bandwidth enhancement process, a super-resolution process, a noise reduction (NR) process and/or an image stabilization process) and/or an enlargement process (electronic zooming process). Further, the image processing unit 5061 performs a detection process for an image signal in order to perform AE, AF and AWB.

The image processing unit 5061 includes a processor such as a CPU or a GPU, and when the processor operates in accordance with a predetermined program, the image processes and the detection process described above can be performed. It is to be noted that, where the image processing unit 5061 includes a plurality of GPUs, the image processing unit 5061 suitably divides information relating to an image signal such that image processes are performed in parallel by the plurality of GPUs.

The control unit 5063 performs various kinds of control relating to image picking up of a surgical region by the endoscope 5001 and display of the picked up image. For example, the control unit 5063 generates a control signal for controlling driving of the camera head 5005. Thereupon, if image pickup conditions are inputted by the user, then the control unit 5063 generates a control signal on the basis of the input by the user. Alternatively, where the endoscope 5001 has an AE function, an AF function and an AWB function incorporated therein, the control unit 5063 suitably calculates an optimum exposure value, focal distance and white balance in response to a result of a detection process by the image processing unit 5061 and generates a control signal.

Further, the control unit 5063 controls the display apparatus 5041 to display an image of a surgical region on the basis of an image signal for which image processes have been performed by the image processing unit 5061. Thereupon, the control unit 5063 recognizes various objects in the surgical region image using various image recognition technologies. For example, the control unit 5063 can recognize a surgical tool such as forceps, a particular living body region, bleeding, mist when the energy treatment tool 5021 is used and so forth by detecting the shape, colour and so forth of edges of the objects included in the surgical region image. The control unit 5063 causes, when it controls the display unit 5041 to display a surgical region image, various kinds of surgery supporting information to be displayed in an overlapping manner with an image of the surgical region using a result of the recognition. Where surgery supporting information is displayed in an overlapping manner and presented to the surgeon 5067, the surgeon 5067 can proceed with the surgery more safety and certainty.

The transmission cable 5065 which connects the camera head 5005 and the CCU 5039 to each other is an electric signal cable ready for communication of an electric signal, an optical fibre ready for optical communication or a composite cable ready for both of electrical and optical communication.

Here, while, in the example depicted, communication is performed by wired communication using the transmission cable 5065, the communication between the camera head 5005 and the CCU 5039 may be performed otherwise by wireless communication. Where the communication between the camera head 5005 and the CCU 5039 is performed by wireless communication, there is no necessity to lay the transmission cable 5065 in the surgery room. Therefore, such a situation that movement of medical staff in the surgery room is disturbed by the transmission cable 5065 can be eliminated.

**An** example of the endoscopic surgery system 5000 to which the technology according to an embodiment of the present disclosure can be applied has been described above. It is to be noted here that, although the endoscopic surgery system 5000 has been described as an example, the system to which the technology according to an embodiment of the present disclosure can be applied is not limited to the example. For example, the technology according to an embodiment of the present disclosure may be applied to a soft endoscopic system for inspection or a microscopic surgery system. Indeed, the technology may be applied to a surgical microscope for conducting neurosurgery or the like.

The technology according to an embodiment of the present disclosure can be applied suitably to the CCU 5039 from among the components described hereinabove. Specifically, the technology according to an embodiment of the present disclosure is applied to an endoscopy system, surgical microscopy or medical imaging. By applying the technology according to an embodiment of the present disclosure to these areas, blood flow in veins, arteries and capillaries may be identified. Further, objects may be identified and the material of those objects may be established. This reduces the risk to the patient's safety during operations.

Furthermore, it will be appreciated that the technology of the present disclosure may be applied more generally to any kind of imaging device (including industrial imaging devices, for example).

Differentiation between different types of tissues is particularly advantageous for medical imaging, such as in a surgical or clinical environment. **In** particular, differentiation between different types of anatomical structures, such as between healthy tissue and tumorous types of tissue, is of high importance to a surgeon. One approach for improved differentiation of images is based on fluorescence or radioactive marker substances. Approaches such as this support visibility enhancement of certain organic structures pointing to pathologies. However, such an approach is not well suited to differentiation in a real-time environment and requires the fluorescence or radioactive markers to be provided prior to detection/image capture.

Therefore, as noted above, it is desired that an imaging system is provided which can address a number of problems related to the optical performance of imaging devices (such as the medical imaging device described with reference to Figures 1 and 2 of the present disclosure). In particular, an imaging device (that is, an image capture device) which is able to provide an improved accuracy and robustness of discrimination between objects within the scene in a substantially real time environment is desired.

Hence, an image capture device, an endoscope system, an image capture method and a computer program product are provided in accordance with embodiments of the present disclosure.

### <Image Capture Device>

Figure 3 illustrates an image capture device 3000 in accordance with embodiments of the disclosure.

The image capture device 3000 comprises a first beam splitter 3002, a first image sensor circuitry 3004, a second image sensor circuitry 3006 and a polarization unit 3008.

The first beam splitter 3002 is configured to split incident light of a predetermined polarization state P along a first and second path (P1 and P2 respectively) in accordance with a wavelength of the light, wherein light of a first wavelength range is split onto the first path P1 and light of a second wavelength range is split onto the second path P2.

The first image sensor circuitry 3004 of the image capture device 3000 is configured to receive light on the first path P1, the first image sensor circuitry 3004 having a plurality of image sensing regions, wherein each of the image sensing regions is configured to be sensitive to light of a predetermined wavelength within the first wavelength range.

The second image sensor circuitry 3006 of the image capture device 3000 is configured to receive light on the second path P2, the second image sensor circuitry 3006 having a plurality of image sensing regions, wherein each of the image sensing regions is configured to be sensitive to light of a certain polarization state.

Finally, the first polarization unit 3008 of the image capture device 3000 is configured to receive incident light of the predetermined polarization state P and provide light of a plurality of polarization states P', wherein the polarization unit 3008 is arranged on the second path P2 between the first beam splitter 3002 and the second image sensor circuitry 3006.

With the image capture device of the present disclosure, parallel acquisition of spectral and polarization images is ensured by the provision of the first and second image capture circuitry 3004 and 3006 configured on the optical paths P1 and P2 of the first beam splitter 3002. Furthermore, because the first image capture circuitry 3004 has a number of image sensing regions, a plurality of different wavelengths can be analysed in a single image frame (i.e. a single shot or instance of time).

Likewise, because the second image capture circuitry has a number of image sensing regions, the image capture device 3000 can analyse a plurality of polarization states in a single image frame (i.e. a single shot or instance of time).

The combination of two modalities (i.e. a number of spectral wavelengths from the first image capture circuitry 3004 and a number of polarization images from the second image capture circuitry 3006) allows an improved object discrimination performance, because the different imaging modalities provide further information of the objects in the scene but do not interfere with each other during image capture. This further reduces input feature complexity and dimensionality allowing for faster acquisition of images.

Furthermore, as the different images are captured simultaneously using the different image sensing regions of the first and second image sensor circuitry, the improvements in the object discrimination is achieved in a substantially real time environment.

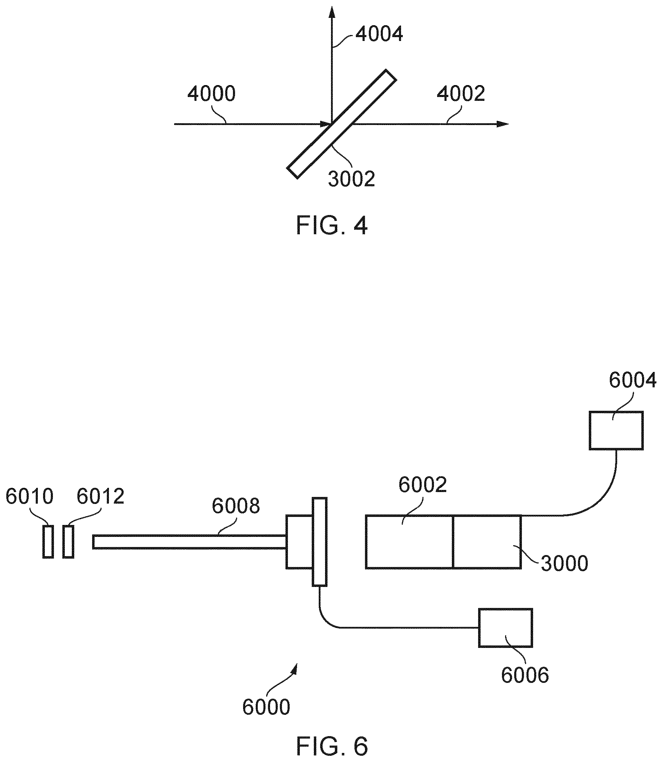

The image capture device of the present disclosure will now be described in more detail with reference to Figures 4 to 11 of the present disclosure.

### <Beam Splitter>

As described with reference to Figure 3 of the present disclosure, the image capture device 3000 comprises a beam splitter 3002.

An example beam splitter 3002 is illustrated with reference to Figure 4 of the present disclosure. That is, Figure 4 illustrates an example configuration of a beam splitter in accordance with embodiments of the disclosure.

In the example of Figure 4, incident light 4000 is intercepted by the first beam splitter 3002 of image capture device 3000. Beam splitter 3002 is thus configured to split the incident light 4000 onto a first path and a second path. That is, the beam splitter 3002 is configured such that at least a first portion 4004 of the incident light 4000 is directed onto the first path while at least a second portion 4002 of the incident light 4000 is directed onto the second path of the beam splitter.

In certain examples, the beam splitter 3002 is a non-polarizing element (i.e. an element which does not affect the polarization state of the incident light).

In certain examples, the beam splitter 3002 may be configured such that the first potion 4004 of the incident light is light which is reflected from the beam splitter, while the second portion 4002 of the incident light is light which is transmitted from the beam splitter.

Furthermore, in examples, the beam splitter may split the light in accordance with the wavelength of the incident light. That is, consider a specific example whereby the incident light comprises light in the wavelength range of 400 to 1700nm. In this specific example, the beam splitter may be configured such that light of a first wavelength range (e.g. 800-1700nm) is reflected onto the first path such that the first portion of light 4004 is light in the wavelength range of 800-1700nm. Then, in this example, the transmitted light which forms the second portion of light is light in the wavelength range 400nm-800nm. In other words, the first beam splitter 3002 may be configured as a dichroic beam splitter.

However, it will be appreciated that the first beam splitter 3002 of the image capture device 3000 is not particularly limited in this regard. That is, while the beam splitter 3002 has been described, with reference to Figure 4 of the present disclosure, as transmitting and reflecting light of specific wavelength ranges, the present disclosure is not particularly limited in this regard. In fact, the first beam splitter 3002 of the image capture device 3000 is not particularly limited in this regard provided that at least a first portion of light is split onto a first path and at least a second portion of light is split onto a second path.

Furthermore, the beam splitter 3002 is not particularly limited to any specific type of beam splitter and may be configured as either a cube beam splitter or a plate beam splitter, for example.

However, it will be appreciated that using the beam splitter 3002 within the image capture device 3000 enables the light incident to the image capture device 3000 to be split onto two distinct paths, which thus facilitates the acquisition of plurality of polarization states and a plurality of spectral wavelengths of the light in a single image frame. Moreover, use of the beam splitter in this manner enables the size of the image capture device 3000 to be reduced.

### <Polarization Unit>

As described with reference to Figure 3 of the present disclosure, image capture device 3000 further comprises a polarization unit 3008. The polarization unit 3008 is configured on the second optical path of the beam splitter 3002 such that it is arranged between the beam splitter 3002 and the second image capture circuitry 3006.

Consider a situation where the image capture device 3000 is used in order to obtain images of a scene. Here, the scene is illuminated with light of a first polarization state. Thus, image capture device 3000 receives light of the first polarization state. The beam splitter 3002 of image capture device 3000 then splits this light onto the first and second paths as described with reference to Figures 3 and 4 of the present disclosure. In fact, because the beam splitter, in certain examples, is a non-polarizing element, the light of the first and second path is also light of the first polarization state.

As such, a polarization unit 3008 is provided between the beam splitter 3002 and the second image capture circuitry 3006 on the second path of the beam splitter. Thus, the light of the second path of the beam splitter encounters the polarization unit 3008 before it reaches the second image capture circuitry 3006.

The polarization unit 3008 is configured to convert the light of the predetermined polarization state into light of a number of different polarization states. Therefore, when the light of the second path of the beam splitter 3002 reaches the second image capture circuitry 3006, it comprises light of a number of different polarization states. The second image capture circuitry can then acquire images of the scene in a number of different polarization states.

In some examples, the light of the predetermined polarization state received by the image capture device may be circularly polarized light. In this example, the polarization unit 3008 may be configured to convert the circularly polarized light into a number of different linear polarization states.

For example, in some configurations, the polarization unit 3008 may be a quarter wavelet plate which is configured in order to convert the circularly polarized light into a number of different linear polarization states. In particular, the polarization unit 3008 may, optionally, be configured as a segmented quarter wavelet plate or the like such that circularly polarized light will be converted to a certain linear polarization of light in accordance with a portion of the polarization unit 3008 on which that circularly polarized light is incident.

However, it will be appreciated that the polarization unit 3008 is not particularly limited to these examples. That is, the configuration of the polarization unit will depend, at least in part, on the polarization of the input light (i.e. the light of the first polarization state) and the configuration of the second image capture circuitry.

Nevertheless, it will be appreciated that providing the polarization unit 3008 between the beam splitter 3002 and the second image capture circuitry 3006 on the second path, such that the light of the second path encounters the polarization unit 3008 before the second image capture circuitry 3006, facilitates the acquisition of multispectral-polarimetric images of the scene.

### <Image Capture Circuitry>

As noted above, the image capture device 3000 of the present disclosure comprise a first image capture circuitry 3002 and a second image capture circuitry 3006.

Figure 5A illustrates the image capture circuitry of the present disclosure in more detail. That is, Figure 5A of the present disclosure illustrates an example configuration of image capture circuitry in accordance with embodiments of the disclosure.

In particular, as illustrated in Figure 5A, the first image capture circuitry 3002 may comprise a number of image sensing regions 5000, 5002, 5004 and 5006. Each of the image sensing regions may be configured to be sensitive to light of a particular wavelength of light. In this way, parallel acquisition of images of the scene at a number of different wavelengths can be achieved.

The first image capture circuitry is configured to be sensitive to light in the first wavelength range (being the light which is split onto the first path of the first beam splitter 3002 of image capture device 3000). As such, each of the individual image sensing regions of the first image capture circuitry 3002 is configured to be sensitive to light of a predetermined wavelength within this first wavelength range (i.e. such that each of the image sensing regions individually provides a spectral image of the scene from within that first wavelength range).

Consider the example whereby the first wavelength range is light in the wavelength range of 800nm-1700nm (i.e. short wavelength infrared). In this example, the first image sensing region 5000 may be configured to detect light of 1200nm, the second image sensing region 5002 may be configured to detect light of 1330nm, the third image sensing region 5004 may be configured to detect light of 1560nm. The fourth image sensing region 5006 may be configured to be sensitive to another wavelength within this wavelength range or may, alternatively, may be configured as an neutral density white or optical density grey filter.

Optionally, a filter unit (not shown) may be provided between the first beam splitter 3002 and the first image capture circuitry 3004. This enables each image sensing region of the image capture circuitry to acquire the images of the scene in parallel. In examples, the filter unit may be a filter matrix array.

This array is typically a 2x2 matrix, which enables concatenation of the spatial resolution of imager in the same manner in both dimensions. In some examples, one or more wavelengths may be repeated twice per matrix. That is, in some examples, where only three infrared spectral bands are to be detected one of the infrared spectral bands may be repeated on the matrix. However, in other such examples, the fourth part of the matrix may be used to provide an additional wavelength band (e.g. a wavelength band such as 940nm or a white or optical density grey filter, for example).

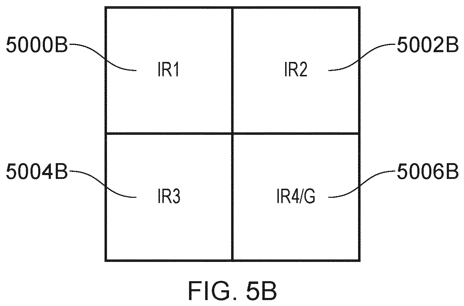

Figure 5B illustrates an example configuration of a filter unit in accordance with embodiments of the disclosure.

More specifically, Figure 5B of the present disclosure illustrates an example 2x2 matrix in accordance with embodiments of the disclosure. This 2x2 matrix may be provided between the first beam splitter 3002 and the first image capture circuitry 3004 as a filter unit. In the example matrix array illustrated in Figure 5B, the sector 5000B is a filter for a first wavelength IR1 (e.g. 1200nm), the sector 5002B is a filter for a second wavelength IR2 (e.g. 1330nm), the third sector 5004B is a filter for a third wavelength IR3 (e.g. 1560nm) and the fourth sector 5006B is provided as a white or optical density gray filter G. However, as explained above, the fourth sector 5006B could be provided as a filter for a fourth wavelength IR4 (e.g. 940nm).

It will be appreciated that the filter matrix array and, more generally, the filter unit, of the present disclosure is not particularly limited to the above described example. In particular, the filter matrix array is not particularly limited to the wavelengths and configuration which have been described with reference to Figure 5B of the present disclosure.

Moreover, the first image capture circuitry of the image capture device 3000 can be provided without the filter unit. **In** this case, the image sensing regions of the image capture circuitry may sequentially acquire the images of the scene (i.e. the multispectral images). Nevertheless, sequential acquisition of the multispectral images can still be performed by the image capture device in parallel with the acquisition of the polarization images (by the second image capture circuitry) such that the image capture device 3000 enables capture of multispectral-polarimatric images for object discrimination in a substantially real time environment.

**In** this way, the first image capture circuitry is able to provide parallel acquisition of four different wavelengths (here, infrared wavelengths) as an image of the scene.

Narrowband imaging - NBI (selective small waveband illumination) can be used to enhance tissue structures contrast. **In** particular, specific difference in reflectance spectra are useful for discrimination of tissue samples. This enables discrimination (or differentiation between) anatomical structures such as bone, fat, nerve and blood vessels. For example, nerves have characteristic reflection spectra (or spectral features) at 1350 and 1500nm. Blood vessels, however, have characteristic reflection spectra (or spectral features) at 1350, 1500 and 1600nm. Therefore, by analysing the different reflection spectra of the image of the scene, it is possible to discriminate between the different tissue or anatomical structures which are present in the scene.

As such, first image capture circuitry configured to capture a number of spectral images of the scene in a wavelength range such as the short wavelength infrared range (i.e. 800nm to 1700nm) is particularly advantageous for the discrimination of anatomical structures in the context of medical imaging and medical imaging devices (or image capture devices). This requires, also, that the beam splitter 3002 is configured to split light of these wavelengths (800nm to 1700nm) onto the first path such that this light can be captured by the first image capture circuitry 3002.

However, it will be appreciated that the first image capture circuitry 3002 of the present disclosure is not particularly limited to this specific example.

That is, while the example of Figure 5A shows only four separate image sensing regions, the present disclosure is not particularly limited in this regard. Indeed, a different number of image sensing regions may be provided on the first image capture circuitry. **In** certain examples, nine different image sensing regions may be provided such that nine different wavelength images (such as nine spectral wavelengths within the infrared region of the spectrum, for example) can be acquired in parallel. The number of image sensing regions can be even more than this. Furthermore, the wavelengths of the image sensing region and the first wavelength range of the present disclosure are not particularly limited to those wavelengths and wavelength ranges which have been described with reference to Figure 5A of the present disclosure. Indeed, the present disclosure is not particularly limited in this regard, provided that the first image capture circuitry is configured to provide spectral images at a number of wavelengths within the first wavelength range (i.e. the wavelength of light split onto the first path by the first beam splitter 3002 of image capture device 3000).

Figure 5A of the present disclosure further illustrates an example configuration of the second image capture circuitry 3006.

In this example, the second image capture circuitry 3006 comprises four different image sensing regions 5008, 5010, 5012 and 5014. Each of the different image sensing regions of the second image sensing circuitry may be configured to be sensitive to a certain polarization of light. For example, the image sensing region 5008 may be sensitive to linear polarized light at a first angle (e.g. a vertical axis with respect to the image capture device 3000), the image sensing region 5010 may be sensitive to linear polarized light at an angle of 45 degrees to the first angle, the image sensing region 5012 may be sensitive to linear polarized light at an angle of 90 degrees to the first angle, and the image sensing region 5014 may be sensitive to linear polarized light at an angle of 135 degrees to the first angle.

In this way, four different linear polarization states of the light can be acquired in parallel by the second image capture circuitry 3006.

For many types of tissue or anatomical structure, the degree of polarization of transmitted or reflected light can be used in order to extract information which can be used in order to enhance the analysis of the tissue or anatomical structure. That is, differences in the degree of polarization of transmitted or reflected light can be used in order to differentiate between the objects in the scene. In particular, the polarization state of the reflected light can be used in order to differentiate between healthy tissue and cancerous tissue. As such, analysis of the polarization state of the light, in addition to the spectral images acquired by the first image capture circuitry, can further facilitate the use of the image capture device 3000 to achieve high accuracy discrimination and identification of objects within the scene.

However, it will be appreciated that the second image capture circuitry is not particularly limited to the example configuration described with reference to Figure 5A of the present disclosure. In particular, the number of image sensing regions are not limited to the number illustrated in Figure 5A and may be more or less than the number of image sensing regions shown. Likewise, the polarization states of the light which are acquired by these image sensing regions are not limited to the specific examples described with this example.

In some examples, at least the first or second image sensor circuitry may be implemented using an image pickup device as described with reference to Figures 1 and 2 of the present disclosure (such as a CMOS sensor or the like). However, any type of image sensor circuity may be used as required depending on the situation to which the embodiments of the disclosure are applied (including, for example, the wavelength of the light which is to be detected).

Nevertheless, it will be appreciated that the first and second image sensing circuitry of the image capture device 3000 are configured such that the image capture device 3000 can analyse a plurality of polarization states and a plurality of spectral wavelengths of the light in a single image frame (i.e. a single image shot or instance of time).

### <Advantageous technical effects>

The image capture device 3000 therefore enables parallel acquisition of spectral and polarization images of the scene by virtue of the dedicated configuration described with reference to Figure 3 of the present disclosure where the first and second image capture circuitry 3004 and 3006 are positioned behind the beam splitter 3002 and, for the second image capture circuitry 3004, behind the polarization unit 3008.

As such, a one frame (one shot) spectral-polarimetric image capture can be achieved with the image capture device 3000. The combination of the two modalities (the spectral images captured by the first image capture circuitry, and the polarization images captured by the second image capture circuitry) reduce input feature complexity and improve the discrimination performance of the image capture device as the image modalities do not interfere with each other.

**In** the context of medical imaging devices (such as endoscopic or laparoscopic imaging devices) the image capture device 3000 thus enables improved visual discrimination, which facilitates identification and differentiation of healthy tissue (such as nerve and blood vessel tissue) against cancerous tissue without the use of fluorescence or radioactive tumour markers.

Hence, the image capture device 3000 is able to provide for improved accuracy and robustness of discrimination between objects within the scene in a substantially real time environment.

### <Endoscope System>

The image capture device 3000 can, in some examples, be configured as part of an endoscope or laparoscope system.

Figure 6 illustrates an example configuration of an endoscope system in accordance with embodiments of the disclosure. Specifically, Figure 6 illustrates an example where the image capture device 3000 is configured as part of an endoscope system (such as a medical endoscope system or the like).

**In** this example, the endoscope system 6000 is shown. The endoscope system 6000 comprises a camera head 6002. The image capture device 3000 of the present disclosure is configured as part of the camera head 6002 of the endoscope system in this example.

The camera head 6002 of the endoscope system is configured such that it is attached to a telescope section 6008. This may, in examples, be a standard endoscope with broadband anti-reflective coatings. In particular, where the endoscope system 6000 is to be used in order to obtain spectral images in the short-wavelength infrared range the telescope section 6008 may be coated with anti-reflective coatings for this wavelength range.

In the endoscope system 6000, a light source 6006 is also provided. This light source 6006 is configured to provide light to illuminate the scene to be imaged. The light source 6006 may be connected by a light cable to the telescope section 6008. The light source 6006 may be configured to produce light in the wavelength range required by the image capture device 3000 and may be configured to generate multiple wavebands.

In this example, the light source 6006 provides unpolarised light. Therefore, polarization elements 6010 and 6012 are provided at the end of the telescope section 6008 in order to convert the unpolarised light from the light source 6006 into light of the predetermined polarization state. In this specific example, the polarization elements 6010 and 6012 comprise a quarter wavelet plate and a linear polarizing ring respectively in order to convert the unpolarised light from the light source 6006 into circularly polarized light. Hence, the scene to be imaged is illuminated with circularly polarized light.

Reflected light from the scene is then acquired by the objective lens of the telescope section 6008 and forms the incident light which is received by the camera head section 6002 (which, as described above, comprises the image capture device 3000).

The image capture device 3000 of the present disclosure thus acquires multispectral-polarimetric images of the scene in the manner described with reference to Figures 3 to 5 of the present disclosure.

A video control unit 6004 is further provided as part of the endoscope system. That is, the endoscope system may further comprise a video control unit configured to control the imaging device to obtain images of the scene.

The video control unit 6004 is connected to the image capture device 3000. In some examples, the connection may be a wired connection. However, in other examples, the video control unit 3004 may be connected to the image capture device 3000 by a wireless connection. The video control unit can therefore obtain the multispectral-polarimetric images of the scene which have been acquired by the image capture device 3000. The video control unit 6004 can then perform further analysis on the images which have been acquired in order to segment an image of the scene in accordance with the different types of objects which are present in the scene.

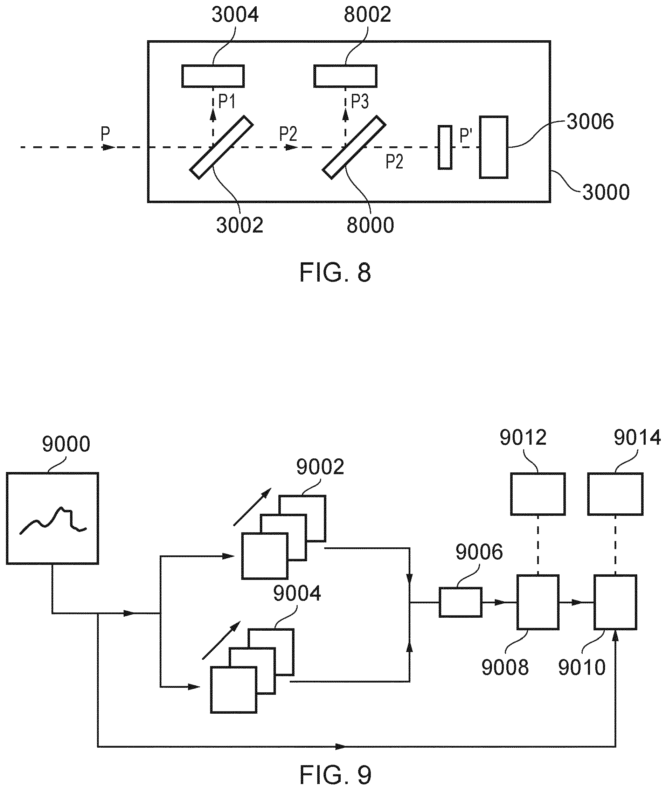

In this manner, the video control unit 6004 of the endoscope system 6000 may be configured to control the acquisition of the images of the scene by the image capture device 3000. Further details regarding the processing performed by the video control unit 6004 will be described with reference to Figure 9 of the present disclosure.

It will be appreciated that while the use of the image capture device 3000 in an endoscope system is described with reference to Figure 6, the present disclosure is not particularly limited in this regard. That is, the image capture device 3000 may be used to acquire images of the scene independently to the endoscope system 6000 and, furthermore, may be used in many other types of imaging systems than the endoscope system 6000 which has been described in this specific example.

### <Light Source>

In the example of Figure 6 of the present disclosure, the endoscope system 6000 is described as containing a light source 6006 which, in combination with the polarization elements 6010 and 6012, produces the light of the first polarization which is required by image capture device 3000.

However, the present disclosure is not particularly limited in this regard. In some optional examples, the light source 6006 may be part of the image capture device 3000 itself.

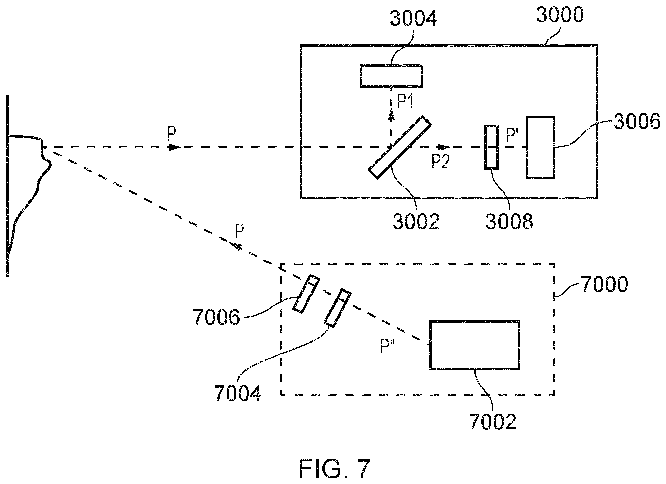

Figure 7 illustrates an example configuration of an image capture device in accordance with embodiments of the disclosure.

In this example, an image capture device 3000 is provided. Image capture device 3000 comprises a beam splitter 3002, a first image capture circuitry 3004, a second image capture circuitry 3006 and a polarization unit 3008. The parts of the image capture device 3000 which are the same as described with reference to Figure 3 of the present disclosure will not be described in detail at this stage, for brevity of disclosure.

Furthermore, a light source unit 7000 is provided as part of the image capture device 3000 and is configured to produce light of the predetermined polarization state.

In this example, the light source unit 7000 comprises a light source 7002 configured to generate unpolarised light in multiple wavebands and a second polarization unit 7004, 7006 configured to convert the unpolarised light to light of the predetermined polarization state. However, the light source unit 7000 is not particularly limited in this regard, and any light source which can produce light of the predetermined polarization state can be used in accordance with embodiments of the disclosure if required.

In a first example, the light source 7002 of light source unit 7000 may comprise an RGB laser and a halogen light source. This enables unpolarised light of the spectral range 400 to 1700nm to be produced. Within the light source 7002, the light emitted from the RGB laser (e.g. light of wavelength range 400nm to 800nm) can be combined with light emitted from the halogen light source (e.g. light in the wavelength range of 800nm to 1700nm) be a light combiner inside the light source to provide a single source of unpolarised light in the desired wavelength range (e.g. 400 to 1700nm).

This unpolarised light is then converted to light of the predetermined polarization state (e.g. circularly polarized light) by the polarization element 7004, 7006 (which may, optionally, comprise a quarter wavelet plate and a linear polarizing ring respectively).

This first example of the light source is particularly advantageous when a filter matrix array (not shown) is provided between the beam splitter 3002 and the first image capture circuitry 3004 of the image capture device 3000.

That is, this example configuration of the light source unit 7000 can be used in order to provide a continuous source of illumination which, coupled with the filter matrix array, enables the parallel acquisition of multispectral images by the individual image sensing regions of the first image capture circuitry 3004. For example, the light source 7002 of the light source unit 7000 then outputs unpolarised P" light of the wavelength range 400 to 1700nm. Polarizing elements 7004, 7006 then convert this light to light of the predetermined polarization state P. Reflected light P of this predetermined polarization state is intercepted by the image capture device 3000 where the beam splitter 3002 splits this light, based on wavelength, onto two distinct paths. The first of these paths P1 may, in this example, comprise light of the wavelength range 800 to 1700nm. When a filter matrix array is provided between the beam splitter 3002 and the first image capture circuitry 3004, this light of the first wavelength range can then be filtered in accordance with the predetermined spectral wavelength corresponding to each image sensing region of the first image capture circuitry. The first image capture circuitry 3004 can therefore acquire, in parallel, multispectral images of the scene in this first wavelength range. At the same time, the second image capture circuitry 3006 can acquire polarization images of the scene from the light of the second path of the beam splitter 3002.

Hence, the use of the light source of this first example facilitates parallel acquisition of the multispectral images of the scene (in combination with the filter matrix array).

In some examples, the light source unit 7000 may be configured to produce light of each of the predetermined wavelengths of the image sensing regions of the first image sensor circuitry in sequence. Then, the first image sensor circuitry may also be configured to acquire the light of the predetermined wavelength from each respective image sensing region in sequence.

In particular, the light source 7002 of the light source unit 7000 may comprise an RGB-LED array and an IR-LED array. The RGB-LED array may be configured to produce light in the wavelength range 400nm to 800nm. Alternatively, a white light LED may be used in order to generate the light of this wavelength range. In contrast, the IR-LED array may be configured to produce light in the wavelength range of 800-1700nm.

In particular, the IR-LED array may comprise a number of LEDs which are configured to produce light of the wavelength of light which is used for multispectral imaging by the first image capture circuitry 3004. That is, the IR-LEDs may produce light at the spectral wavelengths corresponding to each of the image sensing regions of the first image capture circuitry 3004 of the image capture device 3000.

The light from the IR-LED array and the RGB-LEDs may be combined within the light source 7002 by a light combiner such that the light source provides light in the spectral range of 800 to 1700nm. In some examples, the light may be unpolarised light (which is then converted to light of a predetermined polarization state by the polarization element 7004, 7006. In other examples, the light which is produced may itself be polarized (e.g. in the predetermined polarization state). Indeed, in some examples, the light in the wavelength range above a certain wavelength (e.g. 800nm) may be either polarized or unpolarised, since the polarization of the light does not influence the spectral acquisition of that light. As such, in some examples, the light in the first wavelength range (being the light split onto the path P1) which is used for spectral acquisition by the first image capture circuitry can be either polarized or unpolarised light.

This second example configuration of the light source unit 7000 is particularly advantageous when a filter matrix array is not provided between the beam splitter 3002 and the first image capture circuitry 3004.

That is, the IR-LED array may be configured to produce pulsed spectral light in the spectral range of 800nm to 1700nm. That is, the IR-LED array may be configured to produce light at each spectral wavelength required by the first image capture circuitry (e.g. 1200nm, 1330nm and 1560nm) in sequence. Therefore, while a continuous illumination of the scene is provided, the spectral wavelengths which are used in order to illuminate the scene may vary in sequence.

For example, the light source 7002 may first produce light in the spectral range 400-800nm (from the RGB LEDs) and light with a centre wavelength of 1200nm (from a first of the pulsed IR-LEDs). This unpolarised light from the light source 7002 may be converted to polarized light P of the predetermined polarization state by the polarization elements 7004, 7006, before being reflected from a target object in the scene. This reflected light is then intercepted by the image capture device 3000 before being split, based on wavelength, into two paths by the beam splitter 3002. In this example, the first path P1 will then comprise light only of central wavelength 1200nm, while the light of wavelength range 400-800nm (from the RGB LEDs) will be split onto the second path. As such, even without a filter matrix array, the image sensing regions of the first image capture circuitry can acquire a spectral image of the scene at 1200nm.

Then, once the spectral image of the scene at 1200nm has been acquired, the IR-LED may sequentially switch to the production of light at 1330nm (or some other spectral wavelength in the first wavelength range). Accordingly, in the same process as described above, the first image capture circuitry can then acquire a spectral image of the scene at 1330nm.

This process can continue as the light source sequentially pulses through the IR-LED array.

At the same time as the spectral images of the scene are being acquired by the first image capture circuitry, the second image capture circuitry can continue to acquire a plurality of polarization images of the scene.

Accordingly, the pulsed IR-LED array can be used in order to facilitate acquisition of simultaneous multispectral-polarimetric images of the scene in a substantially real time environment even when a filter matrix array is not provided between the beam splitter 3002 and the first image capture circuitry 3004.

Of course, it will be appreciated that the present disclosure is not particularly limited to the specific examples of the light source unit described with reference to Figure 7 of the present disclosure. In particular, the light source unit is not limited to the production of the specific wavelengths of light which have been described with reference to this specific example. Rather, the wavelengths of light which are produced will depend on the configuration of the first image capture circuitry (which, in turn, may depend on the type of image target or scene which is to be imaged). As explained with reference to Figure 6 of the present disclosure, the light source unit can be part of the image capture device 3000 or, alternatively, part of an imaging system (such as the endoscope system) in which the image capture device 3000 is used.

Furthermore, it will be appreciated that, in some examples, a light source device need not specifically be provided at all, provided that the image capture device 3000 can acquire light of the predetermined polarization from the image scene (e.g. where some external illumination of the scene is provided).

### <RGB image capture>

A difficulty with image capture devices which can capture images for differentiation between objects in a scene is that it can often be difficult to implement these image capture devices in a system while still providing an operator (such as a surgeon) a reference image of the scene.

As such, in examples, the image capture device of the present disclosure is able to acquire a reference image of the scene which can be provided to a surgeon in a substantially real time environment in addition to the acquisition of the multispectral-polarimetric images of the scene. The reference image of the scene may be a true colour or RGB image of the scene which can be viewed by the surgeon on an external display unit.

In a first example, the reference image of the scene is acquired by the second image capture circuitry of the image capture device 3000.

Consider, again, the example configuration of Figure 3 of the present disclosure. In this example, the image capture device comprises a beam splitter 3002, a first image capture circuitry 3004, a second image capture circuitry 3006 and a polarization unit 3008.

The first image capture circuitry 3004 comprises a plurality of image sensing regions which are configured to acquire multispectral images of the scene from the first optical path P1 of the beam splitter 3002.

The second image capture circuitry 3006 comprises a plurality of image sensing regions which are configured to acquire a plurality of polarization images of the scene from the second optical path P2 of the beam splitter 3002.

However, in some examples, the second image capture circuitry 3006 may further be configured to capture a reference image of the scene. In particular, in some examples, the second image capture circuitry 3006 may further comprise a number of image sensing regions which are configured in order to capture a reference image of the scene in parallel to the acquisition of the polarization images.

In this regard, the beam splitter 3002 is configured to split the incident light into two paths in accordance with the wavelength of the light. Therefore, in some examples, the light of the second path can be configured to be light in the spectral range of 400nm to 800nm (e.g. light produced by RGB lasers or RGB LED arrays, for example). As such, the light of the second path which reaches the second image capture circuitry may, in some examples, be light in the spectral range of 400nm to 800nm. This light can then be used by the second image capture circuitry 3006 to acquire the reference image of the scene.

In examples, the second image capture circuitry may be provided with a broadband colour filter in order to filter the incident light in accordance with the image sensing regions of the second image capture circuitry which are configured to capture the RGB image of the scene. In some examples, the broadband colour filter may be a Bayer pattern filter.

In this manner, the second image capture circuitry 3006 of the image capture device 3000 may be configured in order to capture an RGB image (reference image) of the scene in parallel to the acquisition of the polarization images of the scene.

This reference image can then be provided (or otherwise displayed) to the operator during operation of the image capture device 3000.

However, in other example configurations of the image capture device 3000, the reference image may be captured by a third image capture circuitry provided in addition to the first and second image capture circuitry within the image capture device 3000.

Consider, now, the example of Figure 8 of the present disclosure. In Figure 8, an example configuration of an image capture device 3000 of the present disclosure is illustrated.

The image capture device 3000 comprises a beam splitter 3002, a first image capture circuitry 3004, a second image capture circuitry 3006 and a polarization unit 3008. These parts of the image capture device 3000 are the same as described with reference to Figure 3 of the present disclosure. Therefore, a detailed explanation of these parts of the image capture device 3000 will not now be provided, for brevity of disclosure.

However, additionally, the image capture device 3000 as illustrated in the example of Figure 8 of the present disclosure further comprises a second beam splitter 8000 and a third image capture circuitry 8002.

In particular, in this example configuration, a second beam splitter 8000 is arranged on the second path P2 of the first beam splitter 3002, such that the light of the second path P2 is split into two distinct paths. The second beam splitter 8000 is arranged between the first beam splitter and the first polarization unit 3008 of the image capture device 3000.

In this example, the second beam splitter 8000 is configured to split the light of the second path between the second path P2 and a third path P3, such that a predetermined quantity of the light of the second path is split onto the third path. However, in contrast to the first beam splitter 3002, the second beam splitter 8000 may not, in examples, be a dichroic beam splitter (that is, it does not split the light in accordance with the wavelength of the light). Rather, the second beam splitter 8000 in examples splits the light of the second path P2 such that a percentage of the light of that path is split onto the third path P3, while a further percentage of the light of that path continues on the second path P2. In some examples, approximately 40% of the light of the second path from the first beam splitter 3002 may continue on the second path P2 while approximately 60% of the light of the second path from the first beam splitter 3002 may be split by the second beam splitter 8000 onto the third path P3. However, the present disclosure is not particularly limited to these specific examples, and different quantities of light may be split between the second P2 and the third path P3 by the second beam splitter 8000 as required in accordance with the situation to which the embodiment of the disclosure is applied.

The third image sensor circuitry is configured to receive light on the third path. That is, the third image sensor circuitry is arranged within the image capture device 3000 such that the light on the third path P3 encounters the third image sensor circuitry.

Similar to the first and second image sensor circuitry described with reference to Figure 3 of the present disclosure, the specific implementation of the third image sensor circuitry is not particularly limited in accordance with embodiments of the disclosure. For example, in some examples, the third image capture circuitry may comprise an image sensor such as that described with reference to Figure 2 of the present disclosure (e.g. a CMOS sensor or the like) or indeed any other type of suitable image pickup circuitry as required (e.g. depending on the wavelength of the light to be detected).

Furthermore, a broadband colour filter array may be provided in front of the third image sensor circuitry. This may be a filter with a Bayer pattern, for example.

In this manner, the third image sensor circuitry being configured to produce a true colour image of the scene.

Furthermore, the light of the second path of the beam splitter 3002 and the beam splitter 8000 continues on the second path P2 and encounters the polarization unit 3008, which converts the light which remains on said second path (being of the predetermined polarization state) to a plurality of different polarization states (such as a plurality of linear polarization states). As such, the second image capture circuitry 3006 is configured to obtain a plurality of polarization images of the scene.

Therefore, the configuration of the image capture device described with reference to Figure 8 of the present disclosure enables parallel acquisition of multispectral-polarimetric images of the scene (e.g. for object differentiation and image segmentation) alongside the acquisition of a reference image (such as an RGB or true colour image of the scene).

Furthermore, the example configuration illustrated in Figure 8 of the present disclosure, with the first, second and third image capture circuitry, enables a higher resolution of reference image and spectrum polarization images to be acquired than compared to the configuration where the second image capture circuitry is configured to acquire both the polarization images and the reference image. Therefore, this example configuration is particularly advantageous when high resolution images of the scene are desired.

It will be appreciated that the present disclosure is not particularly limited to the specific example described with reference to Figure 8 of the present disclosure (that is, different wavelengths of light may be used, for example). Moreover, the image capture device 3000 as illustrated in the example of Figure 8 of the present disclosure may be used in any image capture system (e.g. the endoscope system) as described with reference to Figure 6 of the present disclosure. Furthermore, the example configuration of the image capture device described with reference to Figure 8 of the present disclosure may be combined with a light source unit such as the light source unit described with reference to Figure 7 of the present disclosure for the acquisition of the images of the scene.

### <Image Processing>

The image capture device 3000 of the present disclosure may be used in order to acquire images of the scene which can be used in order to provide a surgeon with an improved accuracy and robustness of discrimination between objects within the scene in a substantially real time environment.

Figure 9 illustrates an example image acquisition process in accordance with embodiments of the disclosure.

Specifically, in this regard, Figure 9 of the present disclosure illustrates an image acquisition and processing chain for images acquired using the image capture device 3000 of the present disclosure.

The processing performed in this example may be performed by a video control unit 6004 such as that described with reference to Figure 6 of the present disclosure, for example.

A tissue sample 9000 to be imaged by a surgeon is illustrated in Figure 9. The surgeon may be imaging this tissue sample as part of a surgical procedure, surgical intervention or the like. In order to obtain an image of the tissue sample 9000, the surgeon may use an endoscope system such as that described with reference to Figure 6 of the present disclosure. An image capture device 3000 is included as part of the camera head 6002 of this endoscope device.

The tissue sample 9000 which is being imaged by the surgeon may comprise a number of different types of tissue or a number of different anatomical structures. As such, it can be difficult for the surgeon to differentiate between the different types of tissue or different anatomical structures which are present in the image when using a standard image capture device. This may limit the surgeons ability to perform any required surgical procedure.

However, because the surgeon is using an endoscope system comprising an image capture device 3000 of the present disclosure, parallel acquisition of multispectral-polarimetric images of the scene in a substantially real time environment is possible which enables an improved identification of objects within the scene to be performed and thus a more accurate segmentation of the image.

That is, in this example, the first image capture circuitry 3004 is configured to acquire a plurality of multispectral images 9004 of the scene. Specifically, these images may be short wavelength infra-red spectral images of the scene, based on the reflectance spectra of the tissue sample under the predetermined illumination. As such, these multispectral images may include images at example wavelengths of e.g. 1200nm, 1330nm and 1560nm.

Furthermore, in this example, the second image capture circuitry 3006 is configured to acquire a plurality of polarization images 9002 of the scene. These images of the scene may comprise, for example, a plurality of linear polarization images of the scene corresponding to the different configurations of the image sensing regions of the second image capture circuitry 3006.

Finally, a RGB image 9010 of the scene is also acquired by the image capture device 3000. This may be acquired optionally by either the second image capture circuitry 3006 or, alternatively, by the third image capture circuitry 8002 described with reference to Figure 8 of the present disclosure.

It will be appreciated that the multispectral images 9004, the polarization images 9002 and the RGB image 9010 of the scene may be acquired in parallel by the image capture device 3000 in a substantially real time environment. In other words, these images can be obtained in a single image shot by image capture device 3000 and thus provide a coherent multi-modal set of images of the tissue 9000 at the time of image capture.

At 9006, the video control unit 6004 performs certain processing in order to combine the different images which have been acquired by the image capture circuitry of the image capture device 3000. In particular, the video control unit 6004 combines the multispectral images acquired by the first image capture circuitry 3004 and the polarization images acquired by the second image capture circuitry 3006.

It will be appreciated that the manner of the processing performed by the video control unit 6004 in order to combine these images is not particularly limited in accordance with embodiments of the disclosure. However, the video control unit 6004 is configured to combine these images (which each provide a different modality of information regarding the scene) in order to perform object identification and differentiation such that the image of the scene can be segmented.

In some examples, the video control unit 6004 may be configured to perform this processing to combine the different images which have been acquired using a trained model. That is, a model (such as a deep learning model) may be trained on a number of training images for the identification of certain objects or types of tissue within the scene. The training images may be based on simulated images of the scene or, alternatively, may include historic images where certain objects within the scene have been pre-identified by a user. For example, the trained model may have been trained on a large set of training images (including images of the same spectral wavelengths and same polarization states which are acquired by the image capture device) which comprise certain objects of the scene. As such, the trained model is able to utilize the images of the scene acquired by the image capture device in order to differentiate between the objects present in the scene and perform subsequent image segmentation.

For example, in the specific context of this example, the trained model may be trained to identify nerves in the tissue sample based on the reflectance spectra of those nerves at wavelengths of 1350 and 1500nm. In contrast, lymph nodes may be identified by the trained model based on their characteristic reflectance spectra at 1370 and 1570nm. Likewise, the trained model may be trained to differentiate between cancerous tissue (such as a tumour) or healthy tissue based on specific variations in the polarization states of the images. In particular, the trained model may be used in order to identify lymph nodes and lymph vessels (including detecting sentinel lymph nodes during tumour tissue resection, for example). Precursor lesion of pancreatic ductal carcinoma and developed ductal carcinoma and intestine tumours can also be identified by the trained model in this manner (i.e. using the multi-modal images which have been acquired by the image capture device).

Advantageously, by utilizing the different imaging modalities which have been acquired in parallel, a more robust segmentation of anatomical structures can be performed by the video control unit 6004, even under varying illumination and in-situ conditions. Moreover, combining the different imaging modalities reduces the input feature complexity and dimensionality as the different imaging modalities do not interfere during the capturing, enabling faster and more computationally efficient image processing to be performed.

Once the different objects (e.g. types of anatomical structures) within the images have been identified, the video control unit 6004 can produce an output image 9012 which is an image of the scene where the different objects within the scene have been highlighted or otherwise identified (e.g. a segmented image of the target tissue). In some examples, this could be a high contrast image of the scene, where the contrast between the different types of objects within the scene has been enhanced by the video control unit in order to show the differentiation between the objects. In other examples, this may be a false colour image of the scene, where different objects within the scene have been highlighted in different colours. For example, a first anatomical structure (e.g. a nerve) may be shown in a first colour while a second anatomical structure (e.g. bone) may be shown in a second different colour.

Furthermore, the RGB image which has been acquired by the image capture device 3000 may be used by the video control unit when producing the output image.

However, the present disclosure is not particularly limited in this regard, and any suitable method for indicating the differentiation between the objects which have been identified in the scene may be used as required.

In some examples, this output image which has been produced may then be shown on an external display unit or the like. This enables an operator of the image device 3000 (e.g. a surgeon or the like) to obtain a substantially real time understanding of the variations in structure of the imaging target.

Furthermore, the RBG image 9014 which has been acquired by the image capture device 3000 can also be provided for external display by the video control unit at step 9010. This ensures that the operator (e.g. the surgeon in this example) is provided with a reference image of the scene acquired at the same time as the multispectral-polarimetric images of the scene.

In this manner, an apparatus or computational device, such as the video control unit 6004 as described with reference to Figure 6 of the present disclosure, may perform image acquisition and processing using the image capture device 3000 in order to provide improved accuracy and robustness of discrimination between objects within the scene in a substantially real time environment.

### <Computer Device>

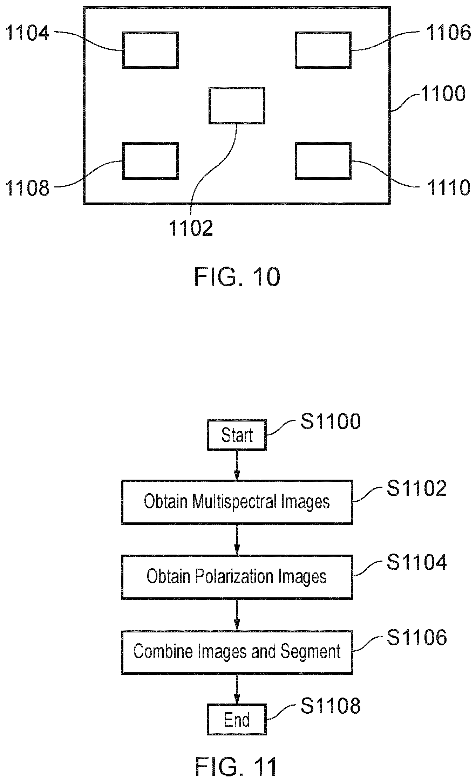

Referring now to Figure 10, an apparatus 1100 according to embodiments of the disclosure is shown. The apparatus 1100 may be an apparatus such as the video control unit 6004 of the endoscope unit 6000 or may be some other apparatus which performs image processing on the images which have been acquired by the image capture device 3000 (such as the image processing described with reference to Figure 9 of the present disclosure, or an image capture method as described with reference to Figure 11 of the present disclosure).

Typically, the apparatus 1100 according to embodiments of the disclosure is a computer device such as a personal computer or a terminal connected to a server. Indeed, in embodiments, the apparatus may also be a server. The apparatus 1100 is controlled using a microprocessor or other processing circuitry 1102. In some examples, the apparatus 1102 may be a portable computing device such as a mobile phone, laptop computer or tablet computing device.

The processing circuitry 1102 may be a microprocessor carrying out computer instructions or may be an Application Specific Integrated Circuit. The computer instructions are stored on storage medium 1104 which maybe a magnetically readable medium, optically readable medium or solid state type circuitry. The storage medium 1104 may be integrated into the apparatus 1100 or may be separate to the apparatus 1100 and connected thereto using either a wired or wireless connection. The computer instructions may be embodied as computer software that contains computer readable code which, when loaded onto the processor circuitry 1102, configures the processor circuitry 1102 to perform a method according to embodiments of the disclosure (such as a method of imaging for a medical imaging device as illustrated with reference to Figure 11 of the present disclosure).

Additionally, an optional user input device 1106 is shown connected to the processing circuitry 1102. The user input device 1106 may be a touch screen or may be a mouse or stylist type input device. The user input device 1106 may also be a keyboard or any combination of these devices.

A network connection 1108 may optionally be coupled to the processor circuitry 1102. The network connection 1108 may be a connection to a Local Area Network or a Wide Area Network such as the Internet or a Virtual Private Network or the like. The network connection 1108 may be connected to a server allowing the processor circuitry 1102 to communicate with another apparatus in order to obtain or provide relevant data. The network connection 1102 may be behind a firewall or some other form of network security.

Additionally, shown coupled to the processing circuitry 1102, is a display device 1110. The display device 1110, although shown integrated into the apparatus 1100, may additionally be separate to the apparatus 1100 and may be a monitor or some kind of device allowing the user to visualise the operation of the system. In addition, the display device 1110 may be a printer, projector or some other device allowing relevant information generated by the apparatus 1100 to be viewed by the user or by a third party.

### <Image Capture Method>

Figure 11 illustrates an image capture method in accordance with embodiments of the disclosure.

The method begins at step S1100 and proceeds to step S1102.

In step S1102, the method comprises obtaining multispectral images, being images of the scene at a plurality of predetermined wavelengths, using the first image sensor circuitry 3004 of an image capture device 3000 of the present disclosure.

The method the proceeds to step S1104 (which can, alternatively, be performed in parallel to step S1102).

In step S1104, the method comprises obtaining a plurality of polarization images of a scene using the second image sensor circuitry 3006 of the image capture device 3000 of the present disclosure.

The method then proceeds to step S1106.

In step S1106, the method comprises combining the polarization images and the multispectral images of the scene to segment the image of the scene into a number of image segments corresponding to a type of object in each part of the image of the scene.

Finally, the method proceeds to and ends with step S1108.

While the method of Figure 11 has been described with reference to obtaining images using an imaging device 3000 of the present disclosure, this same method may also be performed using an endoscope device which comprises the image capture device 3000 (or any other type of imaging device of the present disclosure).

While embodiments of the disclosure have been described in relation to an imaging system for a medical imaging device, it will be appreciated that the claimed invention is not limited to medical imaging (or medical imaging devices), and could, instead, be used in any imaging situation. The imaging system according to embodiments of the disclosure could be employed to effect in an industrial imaging device such as an industrial endoscopic device. For example, embodiments of the disclosure could be used in architectural endoscopy, whereby a scale version of a new building or complex can be correctly viewed from the perspective of a person walking through the architectural creation improving the visualisation, design and construction of proposed buildings.

Embodiments of the disclosure could be used for internal visualisation of works of engineering. For example, an imaging device according to embodiments of the disclosure could be used to view the interior of underground pipe systems, such as water pipes, in order to locate leaks or generally survey the structure. An imaging device according to embodiments of the disclosure could also be used for quality control and internal inspection of other mechanical systems such as turbines and engine components.

Alternatively, embodiments of the disclosure could be used in the security and surveillance industry. For example, an imaging device according to embodiments of the disclosure could be used to conduct surveillance in an area where the presence of a person is restricted, such as in an enclosed area or a very tight space.

In all these applications, an image capture device of the present disclosure may be used in order to capture multispectral-polarimetric images of the scene which facilitate the differentiation between and identification of objects within the scene. It will be appreciated that the above are merely examples of possible industrial applications of an imaging system according to embodiments of the disclosure, and many further applications of the imaging device are possible, as would be apparent to the skilled person when reading the disclosure.

Obviously, numerous modifications and variations of the present disclosure are possible in light of the above teachings. It is therefore to be understood that within the scope of the appended claims, the disclosure may be practiced otherwise than as specifically described herein.

In so far as embodiments of the disclosure have been described as being implemented, at least in part, by software-controlled data processing apparatus, it will be appreciated that a non-transitory machine-readable medium carrying such software, such as an optical disk, a magnetic disk, semiconductor memory or the like, is also considered to represent an embodiment of the present disclosure.

It will be appreciated that the above description for clarity has described embodiments with reference to different functional units, circuitry and/or processors. However, it will be apparent that any suitable distribution of functionality between different functional units, circuitry and/or processors may be used without detracting from the embodiments.

Described embodiments may be implemented in any suitable form including hardware, software, firmware or any combination of these. Described embodiments may optionally be implemented at least partly as computer software running on one or more data processors and/or digital signal processors. The elements and components of any embodiment may be physically, functionally and logically implemented in any suitable way. Indeed the functionality may be implemented in a single unit, in a plurality of units or as part of other functional units. As such, the disclosed embodiments may be implemented in a single unit or may be physically and functionally distributed between different units, circuitry and/or processors.

Although the present disclosure has been described in connection with some embodiments, it is not intended to be limited to the specific form set forth herein. The invention is limited only by the scope of the appended claims.

## Claims

1. Image capture device (3000) comprising:
a first beam splitter (3002) configured to split incident light of a predetermined polarization state (P) along a first (P1) and second path (P2) in accordance with a wavelength of the light, wherein light of a first wavelength range is split onto the first path and light of a second wavelength range is split onto the second path;
first image sensor circuitry (3004) configured to receive light on the first path, the first image sensor circuitry having a plurality of image sensing regions (5000, 5002, 5004, 5006), wherein each of the image sensing regions is configured to be sensitive to light of a predetermined wavelength within the first wavelength range;
second image sensor circuitry (3006) configured to receive light on the second path, the second image sensor circuitry having a plurality of image sensing regions (5008, 5010, 5012, 5014), wherein each of the image sensing regions is configured to be sensitive to light of a certain polarization state; and
a first polarization unit (3008) configured to receive incident light of the predetermined polarization state and provide light of a plurality of polarization states (P'), wherein the polarization unit is arranged on the second path between the first beam splitter and the second image sensor circuitry.

2. The image capture device according to claim 1, wherein the first image sensor circuitry is a multispectral image sensor comprising at least four image sensing regions.

3. The image capture device according to claim 1, wherein the second image sensor circuitry is polarization image sensor comprising at least four image sensing regions.

4. The image capture device according to claim 1, wherein, the image capture device further comprises a filter unit configured between the beam splitter and the first image sensor circuitry along the first path;
the filter unit is configured to filter the light into the plurality of predetermined wavelengths of the first wavelength; and
wherein the first image sensor circuitry is configured to acquire the light of each predetermined wavelength in parallel from each of the respective image sensing regions.

5. The image capture device according to claim 4, wherein the filter unit comprises a filter matrix array.

6. The image capture device according to claim 1, wherein the second sensor circuitry is further configured to acquire a true colour image of the scene.

7. The image capture device according to claim 1, wherein the first polarization unit is a quarter wavelet plate.

8. The image capture device according to claim 1, wherein:
i) the predetermined polarization is circularly polarized light and the plurality of polarization states are a plurality of linear polarization states;
or ii) the certain polarization states of the second image capture circuitry comprise linear polarization states of 0, 45, 90 and 135 degrees with respect to a vertical axis through the second image capture circuitry.

9. The image capture device according to claim 1, wherein the light source unit configured to produce light of each of the predetermined wavelengths of the image sensing regions of the first image sensor circuitry in sequence; and
wherein the first image sensor circuitry is configured to acquire the light of the predetermined wavelength from each respective image sensing region in sequence.

10. The image capture device according to claim 1, further comprising a second beam splitter configured on the second path between the first beam splitter and the first polarization unit;
wherein the second beam splitter is configured to split the light of the second path between the second path and a third path, such that a predetermined quantity of the light of the second path is split onto the third path;
and third image sensor circuitry configured to receive light on the third path, the third image sensor circuitry being configured to produce a true colour image of the scene.

11. An endoscope system (6000) comprising the imaging device (3000) according to any of claims 1 to 10.

12. Computer program product comprising instructions which, when implemented by a computer, cause the computer to perform an imaging method, the image capture method comprising :
obtaining (S1100) multispectral images, being images of the scene at a plurality of predetermined wavelengths, using the first image sensor circuitry of an imaging device according to any of claims 1 to 10 or an endoscope system according to claim 11; and
obtaining (S1104) a plurality of polarization images of a scene using the second image sensor circuitry of the imaging device according to any of claims 1 to 10 or the endoscope system according to claim 11; and
combining (S1106) the polarization images and the multispectral images of the scene to segment the image of the scene into a number of image segments corresponding to a type of object in each part of the image of the scene.

## Patentansprüche

1. Bildaufnahmevorrichtung (3000), umfassend:
einen ersten Strahlteiler (3002), der konfiguriert ist, um einfallendes Licht eines vorbestimmten Polarisationszustands (P) entlang eines ersten (P1) und zweiten Pfads (P2) gemäß einer Wellenlänge des Lichts aufzuteilen, wobei Licht eines ersten Wellenlängenbereichs auf den ersten Pfad aufgeteilt wird und Licht eines zweiten Wellenlängenbereichs auf den zweiten Pfad aufgeteilt wird;
eine erste Bildsensorschaltung (3004), die konfiguriert ist, um Licht auf dem ersten Pfad zu empfangen, wobei die erste Bildsensorschaltung eine Vielzahl von Bilderfassungsregionen (5000, 5002, 5004, 5006) aufweist, wobei jede der Bilderfassungsregionen konfiguriert ist, um für Licht einer vorbestimmten Wellenlänge innerhalb des ersten Wellenlängenbereichs empfindlich zu sein;
eine zweite Bildsensorschaltung (3006), die konfiguriert ist, um Licht auf dem zweiten Pfad zu empfangen, wobei die zweite Bildsensorschaltung eine Vielzahl von Bilderfassungsregionen (5008, 5010, 5012, 5014) aufweist, wobei jede der Bilderfassungsregionen konfiguriert ist, um für Licht eines gewissen Polarisationszustands empfindlich zu sein; und
eine erste Polarisationseinheit (3008), die konfiguriert ist, um einfallendes Licht des vorbestimmten Polarisationszustands zu empfangen und Licht einer Vielzahl von Polarisationszuständen (P') bereitzustellen, wobei die Polarisationseinheit auf dem zweiten Pfad zwischen dem ersten Strahlteiler und der zweiten Bildsensorschaltung angeordnet ist.

2. Bildaufnahmevorrichtung nach Anspruch 1, wobei die erste Bildsensorschaltung ein multispektraler Bildsensor, umfassend mindestens vier Bilderfassungsregionen, ist.

3. Bildaufnahmevorrichtung nach Anspruch 1, wobei die zweite Bildsensorschaltung ein Polarisationsbildsensor, umfassend mindestens vier Bilderfassungsregionen, ist.

4. Bildaufnahmevorrichtung nach Anspruch 1, wobei die Bildaufnahmevorrichtung ferner eine Filtereinheit umfasst, die zwischen dem Strahlteiler und der ersten Bildsensorschaltung entlang des ersten Pfads konfiguriert ist;
wobei die Filtereinheit konfiguriert ist, um das Licht in die Vielzahl von vorbestimmten Wellenlängen der ersten Wellenlänge zu filtern; und
wobei die erste Bildsensorschaltung konfiguriert ist, um das Licht jeder vorbestimmten Wellenlänge aus jeder der jeweiligen Bilderfassungsregionen parallel zu beziehen.

5. Bildaufnahmevorrichtung nach Anspruch 4, wobei die Filtereinheit ein Filtermatrixarray umfasst.

6. Bildaufnahmevorrichtung nach Anspruch 1, wobei die zweite Sensorschaltung ferner konfiguriert ist, um ein Echtfarbbild der Szene zu beziehen.

7. Bildaufnahmevorrichtung nach Anspruch 1, wobei die erste Polarisationseinheit eine Viertelwellenplatte ist.

8. Bildaufnahmevorrichtung nach Anspruch 1, wobei:
i) die vorbestimmte Polarisation zirkular polarisiertes Licht ist und die Vielzahl von Polarisationszuständen eine Vielzahl von linearen Polarisationszuständen ist;
oder ii) die gewissen Polarisationszustände der zweiten Bildaufnahmeschaltung lineare Polarisationszustände von 0, 45, 90 und 135 Grad in Bezug auf eine vertikale Achse durch die zweite Bildaufnahmeschaltung umfassen.

9. Bildaufnahmevorrichtung nach Anspruch 1, wobei die Lichtquelleneinheit konfiguriert ist, um Licht jeder der vorbestimmten Wellenlängen der Bilderfassungsregionen der ersten Bildsensorschaltung nacheinander zu produzieren; und
wobei die erste Bildsensorschaltung konfiguriert ist, um das Licht der vorbestimmten Wellenlänge aus jeder jeweiligen Bilderfassungsregion nacheinander zu beziehen.

10. Bildaufnahmevorrichtung nach Anspruch 1, ferner umfassend einen zweiten Strahlteiler, der auf dem zweiten Pfad zwischen dem ersten Strahlteiler und der ersten Polarisationseinheit konfiguriert ist;
wobei der zweite Strahlteiler konfiguriert ist, um das Licht des zweiten Pfads zwischen dem zweiten Pfad und einem dritten Pfad derart aufzuteilen, dass eine vorbestimmte Menge des Lichts des zweiten Pfads auf den dritten Pfad aufgeteilt wird;
und eine dritte Bildsensorschaltung, die konfiguriert ist, um Licht auf dem dritten Pfad zu empfangen, wobei die dritte Bildsensorschaltung konfiguriert ist, um ein Echtfarbbild der Szene zu produzieren.

11. Endoskopsystem (6000), umfassend die Bildaufnahmevorrichtung (3000) nach einem der Ansprüche 1 bis 10.

12. Computerprogrammprodukt, umfassend Anweisungen, die, wenn sie durch einen Computer implementiert werden, den Computer veranlassen, ein Bildgebungsverfahren durchzuführen, das Bildaufnahmeverfahren umfassend:
Erhalten (S1100) von Multispektralbildern, die Bilder der Szene bei einer Vielzahl von vorbestimmten Wellenlängen sind, unter Verwendung der ersten Bildsensorschaltung einer Bildgebungsvorrichtung nach einem der Ansprüche 1 bis 10 oder eines Endoskopsystems nach Anspruch 11; und
Erhalten (S1104) einer Vielzahl von Polarisationsbildern einer Szene unter Verwendung der zweiten Bildsensorschaltung der Bildgebungsvorrichtung nach einem der Ansprüche 1 bis 10 oder des Endoskopsystems nach Anspruch 11; und
Kombinieren (S1106) der Polarisationsbilder und der Multispektralbilder der Szene, um das Bild der Szene in eine Anzahl von Bildsegmenten zu segmentieren, die einer Art von Objekt in jedem Teil des Bilds der Szene entsprechen.

## Revendications

1. Dispositif de capture d'image (3000) comprenant :
un premier diviseur de faisceau (3002) configuré pour diviser de la lumière incidente d'un état de polarisation prédéterminé (P) le long d'un premier (P1) et d'un deuxième trajet (P2) conformément à une longueur d'onde de la lumière, dans lequel de la lumière d'une première plage de longueur d'onde est divisée sur le premier trajet et de la lumière d'une seconde plage de longueur d'onde est divisée sur le deuxième trajet ;
un premier système de circuits de capteur d'image (3004) configuré pour recevoir de la lumière sur le premier trajet, le premier système de circuits de capteur d'image ayant une pluralité de régions de captage d'image (5000, 5002, 5004, 5006), dans lequel chacune des régions de captage d'image est configurée pour être sensible à de la lumière d'une longueur d'onde prédéterminée au sein de la première plage de longueur d'onde ;
un deuxième système de circuits de capteur d'image (3006) configuré pour recevoir de la lumière sur le deuxième trajet, le deuxième système de circuits de capteur d'image ayant une pluralité de régions de captage d'image (5008, 5010, 5012, 5014), dans lequel chacune des régions de captage d'image est configurée pour être sensible à de la lumière d'un certain état de polarisation ; et
une première unité de polarisation (3008) configurée pour recevoir de la lumière incidente de l'état de polarisation prédéterminé et fournir de la lumière d'une pluralité d'états de polarisation (P'), dans lequel l'unité de polarisation est agencée sur le deuxième trajet entre le premier diviseur de faisceau et le deuxième système de circuits de capteur d'image.

2. Dispositif de capture d'image selon la revendication 1, dans lequel le premier système de circuits de capteur d'image est un capteur d'image multispectral comprenant au moins quatre régions de captage d'image.

3. Dispositif de capture d'image selon la revendication 1, dans lequel le deuxième système de circuits de capteur d'image est un capteur d'image de polarisation comprenant au moins quatre régions de captage d'image.

4. Dispositif de capture d'image selon la revendication 1, dans lequel, le dispositif de capture d'image comprend en outre une unité de filtre configurée entre le diviseur de faisceau et le premier système de circuits de capteur d'image le long du premier trajet ;
l'unité de filtre est configurée pour filtrer la lumière en la pluralité de longueurs d'onde prédéterminées de la première longueur d'onde ; et
dans lequel le premier système de circuits de capteur d'image est configuré pour acquérir la lumière de chaque longueur d'onde prédéterminée en parallèle à partir de chacune des régions de captage d'image respectives.

5. Dispositif de capture d'image selon la revendication 4, dans lequel l'unité de filtre comprend un réseau matriciel de filtres.

6. Dispositif de capture d'image selon la revendication 1, dans lequel le deuxième système de circuits de capteur est configuré en outre pour acquérir une image en couleurs vraies de la scène.

7. Dispositif de capture d'image selon la revendication 1, dans lequel la première unité de polarisation est une lame quart d'onde.

8. Dispositif de capture d'image selon la revendication 1, dans lequel :
i) la polarisation prédéterminée est de la lumière à polarisation circulaire et la pluralité d'états de polarisation sont une pluralité d'états de polarisation linéaire ;
ou ii) les certains états de polarisation du deuxième système de circuits de capture d'image comprennent des états de polarisation linéaire de 0, 45, 90 et 135 degrés par rapport à un axe vertical à travers le deuxième système de circuits de capture d'image.

9. Dispositif de capture d'image selon la revendication 1, dans lequel l'unité de source de lumière est configurée pour produire de la lumière de chacune des longueurs d'onde prédéterminées des régions de captage d'image du premier système de circuits de capteur d'image en séquence ; et
dans lequel le premier système de circuits de capteur d'image est configuré pour acquérir la lumière de la longueur d'onde prédéterminée en provenance de chaque région de captage d'image respective en séquence.

10. Dispositif de capture d'image selon la revendication 1, comprenant en outre un second diviseur de faisceau configuré sur le deuxième trajet entre le premier diviseur de faisceau et la première unité de polarisation ;
dans lequel le second diviseur de faisceau est configuré pour diviser la lumière du deuxième trajet entre le deuxième trajet et un troisième trajet, de telle sorte qu'une quantité prédéterminée de la lumière du deuxième trajet est divisée sur le troisième trajet ;
et troisième système de circuits de capteur d'image est configuré pour recevoir de la lumière sur le troisième trajet, le troisième système de circuits de capteur d'image étant configuré pour produire une image en couleurs vraies de la scène.

11. Système d'endoscope (6000) comprenant le dispositif d'imagerie (3000) selon l'une quelconque des revendications 1 à 10.

12. Produit programme informatique comprenant des instructions qui, lorsqu'elles sont implémentées par un ordinateur, amènent l'ordinateur à mettre en œuvre un procédé d'imagerie, le procédé de capture d'image comprenant :
l'obtention (S1100) d'images multispectrales, étant des images de la scène à une pluralité de longueurs d'onde prédéterminées, à l'aide du premier système de circuits de capteur d'image d'un dispositif d'imagerie selon l'une quelconque des revendications 1 à 10 ou d'un système d'endoscope selon la revendication 11 ; et
l'obtention (S1104) d'une pluralité d'images de polarisation d'une scène à l'aide du deuxième système de circuits de capteur d'image du dispositif d'imagerie selon l'une quelconque des revendications 1 à 10 ou du système d'endoscope selon la revendication 11 ; et
la combinaison (S1106) des images de polarisation et des images multispectrales de la scène pour segmenter l'image de la scène en un nombre de segments d'image correspondant à un type d'objet dans chaque partie de l'image de la scène.
